# EUROPEAN PATENT APPLICATION

(11) **EP 4 289 860 A1**
(43) Date of publication of application: **13.12.2023**
(21) Application number: 21920570.5
(22) Date of filing: 09.08.2021
(51) Int. Cl.: C07K 14/605, C07K 14/575, C07K 19/00, A61K 38/22, A61K 38/26

(54) **USE OF POLYPEPTIDE COMPOUND IN PREVENTION OR TREATMENT OF INFLAMMATORY BOWEL DISEASE AND INTESTINAL FIBROSIS RELATED THERETO**

(30) Priority: 22.01.2021 CN 202110087438
(71) Applicant: Shenzhen Turier Biotech Co., Ltd., Guangdong 518000 (CN)
(72) Inventor: ZHANG, Binzhi, Shenzhen, Guangdong 518000 (CN); LIU, Qi, Shenzhen, Guangdong 518000 (CN)
(74) Representative: SSM Sandmair
(86) International application number: PCT/CN2021/111425
(87) International publication number: WO 2022/156189

(57) **Abstract**

The present invention discloses a use of a polypeptide compound in the preparation of drugs for preventing or treating inflammatory bowel diseases and related intestinal fibrosis, wherein the polypeptide compound comprises a parent peptide represented by the following amino acid sequence: His-Xaa2-Gln-Gly-Thr⁵-Phe-Thr-Ser-Asp-Lys¹⁰-Ser-Lys-Tyr-Leu-Xaa15^{1 5}-Xaa16-Xaa17-Ala-Ala-Gln²⁰-Xaa21-Phe-Xaa23-Xaa24-Trp²⁵-Leu-Xaa2 7-Xaa28-Gly-Gly³⁰-Pro-Ser-Ser-Gly-Xaa35³⁵-Pro-Pro-Pro-Ser. The polypeptide compound can not only reduce serum inflammatory factors, inhibit neutrophil infiltration and alleviate inflammation levels; and the polypeptide compound can also reduce pro-fibrotic factors and downregulate protein expressions of α-SMA and Fibronectin and have significant therapeutic effect on inflammatory bowel disease and related intestinal fibrosis.

## Description

### TECHNICAL FIELD OF THE INVENTION

The present invention belongs to the field of biochemical technology, and specifically the invention relates to a new use of a kind of polypeptide compounds, namely the use of polypeptide compounds in preventing or treating inflammatory bowel diseases and related intestinal fibrosis.

### BACKGROUND OF THE INVENTION

Intestinal fibrosis is a more serious complication of inflammatory bowel diseases (IBD), such as Crohn's disease (CD), ulcerative colitis and other chronic intestinal diseases [World Chinese Journal of Digestology, July 8, 2008; 16 (19): 2137-2143]. Intestinal fibrosis is caused by repeated "damage-rehabilitation" reactions in tissues during inflammatory reactions, especially chronic inflammatory processes, leading to overexposure of various inflammatory cytokines, dysregulation of enzymes degrading extracellular matrix (ECM) and their inhibitors, increased proliferation of fibroblasts and myofibroblasts as well as excessive accumulation of various cellular components and extracellular matrix components [Chinese Journal of Digestion, February 2011, Vol. 31, No. 2].

Crohn's disease (CD) is a chronic non-specific intestinal inflammatory disease with unclear etiology. Intestinal fibrosis is a characteristic manifestation of CD, and intestinal stenosis caused by recurrent onset of intestinal fibrosis is one of the most serious complications of CD. The incidence and prevalence rates of CD are the highest in developed countries such as Europe and North America. However, along with the change of diet structure and lifestyle, the annual incidence rate of Crohn's disease (0.54/100,000) was increasing in Asia in recent years [Torres J, Mehandru S, Colombel JF, Peyrin-Biroulet L. Crohn's disease. Lancet, 2017, 389(10080):1741-1755]. The disease is chronic and recurrent and gradually causes intestinal wall fibrosis, leading to decreased intestinal peristalsis, even intestinal stenosis and bowel obstruction. The incidence of intestinal stenosis is higher than 1/3 in CD patients [Hwang JM, Varma MG. Surgery for inflammatory bowel disease. World J Gastroenterol. 2008, 14(17):2678-2690].

Nowadays, the drugs used to treat Crohn's disease are mainly traditional therapeutic drugs, including glucocorticoids, immune agents or biological agents. The effect of aminosalicylic acid acts on intestinal mucosa and Submucosa and is not better than that of placebo in treating Crohn's disease. Clinically, it is not recommended for treating stenosing Crohn's disease. Glucocorticoids are effective drugs for inducing remission of active Crohn's diseases, but some studies suggest that they may accelerate intestinal stenosis [Lichtenstein GR, Olson A, Travers S, et al. Factors associated with the development of intestinal strictures or obstructions in patients with Crohn's disease. Am J Gastroenterol. 2006, 101(5): 1030-1038.]. Azathioprine can delay the occurrence of fibrous stenosis complications after Crohn's disease surgery [Peyrin-Biroulet L, Deltenre P, Ardizzone S, et al. Azathioprine and 6-mercaptopurine for the prevention of postoperative recurrence in Crohn's disease: a meta-analysis. Am J Gastroenterol. 2009, 104(8):2089-2096]. However, because Azathioprine has a slow onset, it is often used in combination with drugs inducing disease remission during the active phase of the disease; after symptom relief, it is used as a medication to maintain relief and prevent worsening of stenosis or emergence of new stenosis. Anti-tumor necrosis factor preparations are effective drugs for inducing and maintaining remission in patients with Crohn's disease. However, there are relatively few studies on the effectiveness of such drugs for treating stenosing Crohn's disease and the results of some small sample studies in the past are inconsistent [Rieder F, Zimmermann EM, Remzi FH, Sandborn WJ. Crohn's disease complicated by strictures: a systematic review. Gut. 2013, 62(7): 1072-1084.].

Existing drugs can only alleviate inflammation and improve symptoms, but cannot improve intestinal fibrosis, although significant progress has been made in the treatment of CD. Therefore, the development strategy of new drugs to delay or block the progression of fibrosis has important practical significance. Besides the intestine, tissue fibrosis is a chronic disease occurring in such organs as liver, lungs, heart and kidneys. After tissue damage, a series of inflammatory reactions occur at the damaged site, leading to excessive precipitation of extracellular matrix and tissue fibrosis. Currently, the treatment for fibrosis mainly focuses on controlling inflammatory reactions and inhibiting key cellular pathways and protein secretion peptides during the fibrosis process. However, the drugs currently used in clinical practice are still limited and usually only have therapeutic effects on fibrosis in certain tissues. For fibrosis in other tissues, these drugs usually do not have therapeutic effects and have not shown the ability to expand indications so that they can be used for the treatment of other systemic fibrosis diseases, especially intestinal inflammatory bowel diseases.

Currently, drugs that directly combat intestinal fibrosis are not used in clinical practice. Surgical intervention is the internationally recognized treatment method for intestinal fibrosis and stenosis, but frequent intestinal resection surgeries due to easy recurrence may easily lead to short bowel syndrome (SBS) [Van Assche G, Geboes K, Rutgeerts P. Medical therapy for Crohn's disease strictures. Inflamm Bowel Dis 2004; 10:55-60]. Therefore, it has become a focus of attention in this field to explore effective prevention and treatment measures with fewer side effects for intestinal fibrosis.

Nowadays, great challenges have to be faced in the treatment of inflammatory bowel diseases and related intestinal fibrosis. It is urgent to provide a drug, which can significantly treat inflammatory bowel diseases and related intestinal fibrosis.

### SUMMARY OF THE INVENTION

The purpose of the present invention is to provide a polypeptide compounds. Through extensive experimental researches, the inventor of the present invention has proved that the polypeptide compound can reduce serum inflammatory factors, inhibit neutrophil infiltration and alleviate inflammation levels; it can also reduce pro-fibrotic factors, downregulate protein expressions of α-SMA and Fibronectin and have significant therapeutic effect on inflammatory bowel disease and related intestinal fibrosis. Moreover, it has a longer half-life and has never caused an adverse reaction.

Another purpose of the present invention is to provide a use of the above polypeptide compound, which can potentially be used as a new generation drug to prevent or treat inflammatory bowel diseases and related intestinal fibrosis.

In order to achieve the above purposes, the technical solution adopted in the present invention is as follows:
The present invention provides a use of a polypeptide compound in the preparation of drugs for preventing or treating inflammatory bowel diseases and related intestinal fibrosis, wherein the polypeptide compound comprises a parent peptide represented by the following amino acid sequence:
His-Xaa2-Gln-Gly-Thr⁵-Phe-Thr-Ser-Asp-Lys¹⁰-Ser-Lys-Tyr-Leu-Xaa15¹⁵-Xaa16-Xaa17-Ala-Ala-Gln²⁰-Xaa21-Phe-Xaa23-Xaa24-Trp²⁵-Leu-Xaa27-Xaa28-Gly-Gly³⁰-Pro-Ser-Ser-Gly-Xaa35³⁵-Pro-Pro-Pro-Ser,
wherein,
Xaa2 = Aib, Ser or D-Ser;
Xaa15 = Asp or Glu;
Xaa16 = Aib or Glu;
Xaa17 = Lys or Arg;
Xaa21 = Asp or Glu;
Xaa23 = Val or Iva;
Xaa24 = Glu or Gin;
Xaa27 = Leu or Lys;
Xaa28 = Asp, Glu or Ala;
Xaa35 = Ala or Aib.

Preferably, Xaa2 = Aib or D-Ser in the amino acid sequence of the parent peptide.

Preferably, Xaa21 = Asp in the amino acid sequence of the parent peptide.

Preferably, the carboxyl terminal in the amino acid sequence of the parent peptide is unmodified or amino-modified to form a -CONH₂ group.

Moreover, preferably, the side chain of Lys at position 10 or 12 in the amino acid sequence of the parent peptide is linked to a lipophilic substituent via a bridging group, which is one of (PEG)ₘ, (PEG)ₘ-γGlu, (PEG)ₘ-Asp, (Gly)ₓ-(Gly-Ser)_{y}-(Gly)_{z}-, (Gly)ₓ-(Gly-Ser)_{y}-(Gly)_{z}-γGlu and (Gly)ₓ-(Gly-Ser)_{y}-(Gly)_{z}-Asp. The linkage is that the side-chain amino group of Lys at position 10 or 12 forms an amide bond with the carboxyl group of the glycine residue or the (PEG)ₘ-terminal-modified carboxyl group at a terminal of the bridging group and the lipophilic substituent is then connected to the parent peptide; meanwhile, the lipophilic substituent is connected by forming an amide bond between its carboxyl group and the amino group of the bridging group at the other terminal; the lipophilic substituent is CH₃(CH₂)nC(O)- or HOOC(CH₂)nC(O)- and its acyl group forms an amide bond with the amino group in the bridging group;

In the bridging group described in the present invention, m is an integer from 2 to 10; x is an integer from 0 to 5; y is an integer from 1 to 5; z is an integer from 1 to 5. In the lipophilic substituent of the present invention, n is an integer from 14 to 20.

Alternatively, the Lys at position 10 or 12 in the amino acid sequence of the parent peptide can be replaced by HomoLys, Orn, Dap or Dab.

Further preferably, in the amino acid sequence of the parent peptide:
Xaa2 = Aib or D-Ser;
Xaa15 = Glu;
Xaa16 = Aib;
Xaa17 = Lys;
Xaa21 = Asp;
Xaa23 = Val;
Xaa24 = Glu;
Xaa27 = Lys;
Xaa28 = Ala;
Xaa35 = Ala;
   or
Xaa2 = Aib or D-Ser;
Xaa15 = Asp;
Xaa16 = Glu;
Xaa17 = Arg;
Xaa21 = Asp;
Xaa23 = Iva;
Xaa24 = Gin;
Xaa27 =Leu;
Xaa28 = Asp or Glu;
Xaa35 = Ala or Aib.

According to the detailed description of the preferred examples of the present invention, the amino acid sequence of the parent peptide is selected from SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15 and SEQ ID NO. 16.

According to the detailed description of the preferred examples of the present invention, Lys at position 10 or 12 (preferably Lys at position 10) in the amino acid sequence of the parent peptide is linked to the lipophilic substituent via the bridging group to form the following structure:
Lys(PEG₂-PEG₂-γGlu-CO(CH₂)₁₈CO₂H) 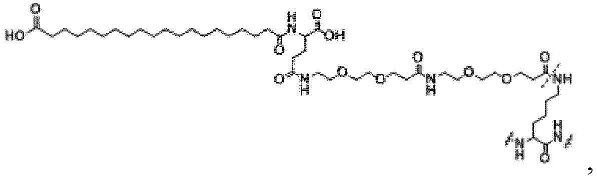 or
Lys(PEG₂-PEG₂-γGlu-CO(CH₂)₁₆CO₂H) 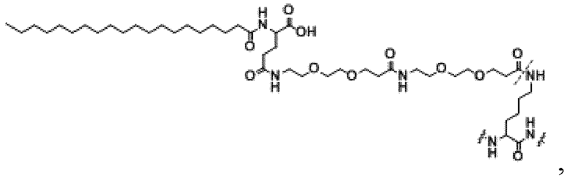 or
Lys(PEG₂-PEG₂-γGlu-CO(CH₂)₁₆CO₂H) 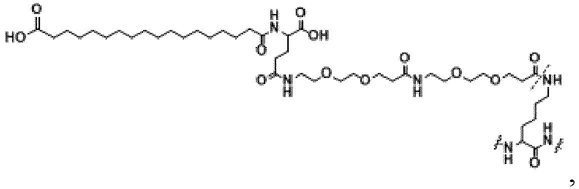 or
Lys(PEG₂-PEG₂-CO(CH₂)₁₈CO₂H) 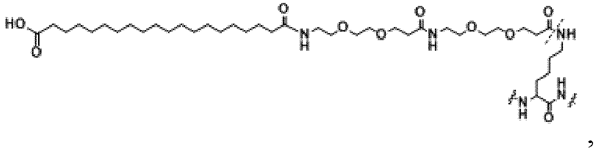 or
Lys(Gly-Gly-Ser-Gly-Ser-Gly-γGlu-CO(CH₂)₁₈CO₂H)

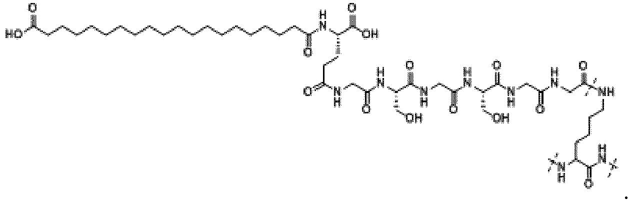

According to the detailed description of the preferred examples of the present invention, the present invention provides any of the following polypeptide compounds:
Compound 1: 
H-Aib-QGTFTSDK(PEG2-PEG2-γGlu-CO(CH2)18CO2H)SKYLE-Aib-KAA
QDFVEWLKAGGPSSGAPPPS-NH2
Compound 2: 
HsQGTFTSDK(PEG2-PEG2-CO(CH2)18CO2H)SKYLE-Aib-KAAQDFVEW
LKAGGPSSGAPPPS-NH2
Compound 3: 
HsQGTFTSDK(PEG2-PEG2-γGlu-CO(CH2)18CO2H)SKYLE-Aib-KAAQDF
VEWLKAGGPSSGAPPPS-NH2
Compound 4: 
HsQGTFTSDK(PEG2-PEG2-γGlu-CO(CH2)16CO2H)SKYLE-Aib-KAAQDF
VEWLKAGGPSSGAPPPS-NH2
Compound 5: 
HsQGTFTSDK(PEG2-PEG2-γGlu-CO(CH2)18CH3)SKYLE-Aib-KAAQDFV
EWLKAGGPSSGAPPPS-NH2
Compound 6: 
HsQGTFTSDK(PEG2-PEG2-γGlu-CO(CH2)18CO2H)SKYLDERAAQDF-Iv
a-QWLLDGGPSSG-Aib-PPPS-NH2
Compound 7: 
HsQGTFTSDK(PEG2-PEG2-γGlu-CO(CH2)18CO2H)SKYLDERAAQDF-Iv
a-QWLLEGGPSSGAPPPS-NH2
Compound 8: 
H-Aib-QGTFTSDK(PEG2-PEG2-γGlu-CO(CH2)18CO2H)SKYLDERAAQD
F-Iva-QWLLDGGPSSG-Aib-PPPS-NH2
Compound 9: 
H-Aib-QGTFTSDK(PEG₂-PEG₂-γGlu-CO(CH₂)₁₆CO₂H)SKYLDERAAQD
F-Iva-QWLLDGGPSSG-Aib-PPPS-NH₂
Compound 10: 
H-Aib-QGTFTSDK(PEG2-PEG2-γGlu-CO(CH2)18CH3)SKYLDERAAQDF-
Iva-QWLLDGGPSSG-Aib-PPPS-NH2
Compound 11: 
H-Aib-QGTFTSDK(PEG2-PEG2-γGlu-CO(CH2)18CH3)SKYLDERAAQDF-
Iva-QWLLDGGPSSG-Aib-PPPS-NH2
Compound 12: 
HsQGTFTSDK(PEG2-PEG2-γGlu-CO(CH2)18CO2H)SKYLDERAAQDF-Iv
a-QWLLDGGPSSG-Aib-PPPS-NH2
Compound 13: 
HsQGTFTSDK(GGSGSG-γGlu-CO(CH2)18-COOH)SKYLE-Aib-KAAQDF
VEWLKAGGPSSGAPPPS
Compound 14: 
HsQGTFTSDK(GGSGSG-γGlu-CO(CH2)18-COOH)SKYLE-Aib-KAAQD
FVEWLKAGGPSSGAPPPS-NH2
Compound 15: H-Aib-QGTFTSDK(GGSGSG-γGlu-CO(CH2)18-COOH)SKYLDERAAQ DF-Iva-QWLLDGGPSSG-Aib-PPPS
Compound 16:

H-Aib-QGTFTSDK(GGSGSG-γGlu-CO(CH2)18-COOH)SKYLDERAAQ
DF-Iva-QWLLDGGPSSG-Aib-PPPS-NH2.

The present invention also provides a composition comprising the polypeptide compound described herein.

Preferably, the composition is a pharmaceutical composition also comprising a pharmaceutically acceptable carrier or excipient.

The present invention further provides a use of the composition in the preparation of drugs for preventing or treating intestinal fibrosis.

The technical solution of the present invention is described in detail as follows.

The first aspect of the present invention is to provide a polypeptide compound comprising a parent peptide represented by the following amino acid sequence: 
His-Xaa2-Gln-Gly-Thr-Phe-Thr-Ser-Asp-Lys-Ser-Lys-Tyr-Leu-Xaa15-Xaa16-X
aa17-Ala-Ala-Gln-Xaa21-Phe-Xaa23-Xaa24-Trp-Leu-Xaa27-Xaa28-Gly-Gly-Pr
o-Ser-Ser-Gly- Xaa35-Pro-Pro-Pro-Ser-COR1
wherein, R₁ = -NH₂ or -OH;
Xaa2 = Aib, Ser or D-Ser;
Xaa15 = Asp or Glu;
Xaa16 = Aib or Glu;
Xaa17 = Lys or Arg;
Xaa21 = Asp or Glu;
Xaa23 = Val or Iva;
Xaa24 = Glu or Gin;
Xaa27 = Leu or Lys;
Xaa28 = Asp, Glu or Ala;
Xaa35 = Ala or Aib.

The side chain of Lys at position 10 or 12 in the amino acid sequence of the parent peptide is linked to a lipophilic substituent via a bridging group.

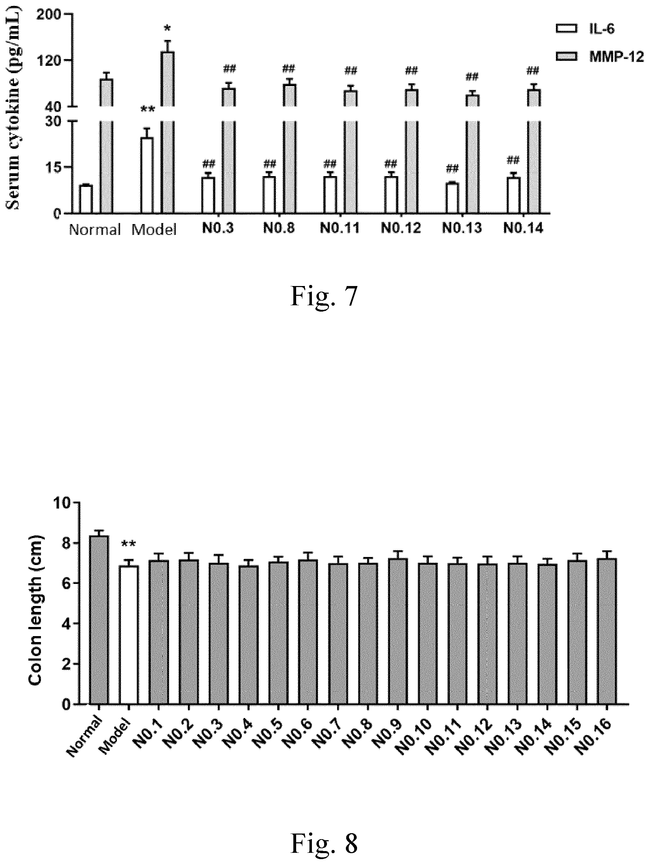

The bridging group comprises (PEG)ₘ or (PEG)ₘ-γGlu or (PEG)ₘ-Asp; it may also comprise (Gly)ₓ-(Gly-Ser)_{y}-(Gly)_{z}- or (Gly)ₓ-(Gly-Ser)_{y}-(Gly)_{z}-γGlu or (Gly)ₓ-(Gly-Ser)_{y}-(Gly)_{z}-Asp. The linkage is that: the side-chain amino group of Lys at position 10 or 12 forms an amide bond with the carboxyl group of the glycine residue or the (PEG)m-terminal-modified carboxyl group at a terminal of the bridging group and is then connected to the parent peptide; meanwhile, the lipophilic substituent is connected by forming an amide bond between its carboxyl group and the amino group of the bridging group at the other terminal. The Lys at position 10 or 12 connected by the lipophilic substituent may be replaced by HomoLys, Orn, Dap or Dab. Wherein, m is an integer from 2 to 10; x is an integer from 0 to 5; y is an integer from 1 to 5; z is an integer from 1 to 5. The lipophilic substituent is selected from CH₃(CH₂)ₙCO- or HOOC(CH₂)ₙCO-, wherein n is an integer from 14 to 20 and the linkage is shown in Fig. 7 and Fig. 8. Fig. 7 is an exemplary modification method of Lys side chain in the parent peptide of the compound of the present invention and Fig. 8 is another exemplary modification method of Lys side chain in the parent peptide of the compound of the present invention.

In the full text of the Specification of this Application, conventional three-letter codes are used to represent natural amino acids and the recognized three-letter codes are used to represent other amino acids, e.g. Aib (2-aminoisobutyric acid) and Orn (ornithine). The compound in the present invention is based on the theory that the intramolecular bridges may stabilize the helical structure of the molecule, thereby improving the efficacy and/or selectivity of polypeptide compounds.

The compound of the present invention is based on the theory that the lipophilic substituent can combine with albumin in the blood to protect the compound proposed in the present invention from enzymatic degradation, thereby improving the half-life of the compound.

The inflammatory bowel diseases and related intestinal fibrosis mentioned of the present invention include Crohn's disease, colitis, intestinal fibrosis caused by Crohn's disease, and intestinal fibrosis caused by colitis.

Another aspect of the present invention is to provide a pharmaceutical composition comprising a polypeptide compound proposed in the present invention, and the pharmaceutical composition is made by adding pharmaceutically acceptable carriers and/or excipients with the polypeptide compound as the active ingredient.

Yet another aspect of the present invention is to a pharmaceutical use for the polypeptide compound proposed in the present invention. The results of animal experiments have shown that the polypeptide compound proposed in the present invention has inhibitory effects on intestinal inflammation and intestinal fibrosis and can be used as a drug for treating Crohn's disease.

The parent peptide of the polypeptide compound mentioned in the present invention is a homologous polypeptide. The homologous polypeptide of the present invention refers to that the polypeptide originally has the amino acid sequence of oxyntomodulin (OXM), glucagon-like peptide (GLP-1), exenatide or glucagon, but one or more amino acid residues have been substituted by different amino acid residues. Moreover, these amino acid residues are conservative with each other and the obtained polypeptide can be used to implement the present invention.

Those skilled in the art can appreciate that the pharmaceutical composition of the present invention is suitable for various administration routes, such as oral administration, percutaneous administration, intravenous administration, intramuscular administration, topical administration and intranasal administration. According to the used administration route, the pharmaceutical composition containing the polypeptide derivative of the present invention can be formulated into various suitable dosage forms, which comprises an effective dose of at least one polypeptide compound proposed in the present invention and at least one pharmaceutically acceptable pharmaceutical carrier. Examples of suitable dosage forms are tablets, capsules, sugar-coated tablets, granules, oral liquid, syrup, ointment and paste for the skin surface, aerosol, nasal spray and sterile solution for injection.

The pharmaceutical composition comprising the polypeptide compound proposed of the present invention may be prepared into solution or lyophilized powder for parenteral administration. Before use, an appropriate solvent or other pharmaceutically acceptable carrier can be added to re-prepare the powder, and liquid formula is generally buffer, osmotic solution and aqueous solution.

The dosage of the pharmaceutical composition of the present invention may vary in a wide range and can be easily determined by those skilled in the art according to certain factors, such as the type of the disease, the severity of the disease, the patient's body weight, the dosage form, the administration route and so forth.

Compared to the prior art, the present invention has the following advantages:
1) The polypeptide compound of the present invention has better biological activity, can significantly reduce serum inflammatory factors, inhibit neutrophil infiltration and alleviate inflammation levels; It can also reduce pro-fibrotic factors and downregulate protein expressions of α-SMA and Fibronectin;
2) The polypeptide compound of the present invention not only has the pharmacodynamic effect in treating complications of diabetic nephropathy and other diabetes, and also has obvious therapeutic effect on inflammatory bowel diseases and related intestinal fibrosis, can prevent and treat acute and chronic colitis, can alleviate inflammation and reduce the level of intestinal fibrosis during the treatment process, and has more advantages in the clinical use in treating Crohn's disease, colitis, fibrosis caused by Crohn's disease, and fibrosis caused by colitis. Clinically, the polypeptide compound of the present invention has better therapeutic effect on inflammatory bowel diseases and related intestinal fibrosis than other drugs;
3) The polypeptide compound of the present invention exhibits significantly extended half-life and stability in drug pharmacokinetic experiments;
4) The polypeptide compound of the present invention has high synthesis yield, good stability, easy scale-up production, and low cost.

In the specific examples, the present invention relates to the following polypeptide compounds, which have the following sequence and side chain modification results:
Compound 1 (SEQ ID NO. 1): His-Aib-Gln-Gly-Thr-Phe-Thr-Ser-Asp-Lys(PEG2-PEG2-γGlu-CO(CH2)18CO2H) 
-Ser-Lys-Tyr-Leu-Glu-Aib-Lys-Ala-Ala-Gln-Asp-Phe-Val-Glu-Trp-Leu-Lys-Ala
-Gly-Gly-Pro-Ser-Ser-Gly-Ala-Pro-Pro-Pro-Ser-NH2;
 
H-Aib-QGTFTSDK(PEG2-PEG2-γGlu-CO(CH2)18CO2H)SKYLE-Aib-KAAQDF
VEWLKAGGPSSGAPPPS-NH2;
Compound 2 (SEQ ID NO. 2): 
His-(D-Ser)-Gln-Gly-Thr-Phe-Thr-Ser-Asp-Lys(PEG2-PEG2-CO(CH2)18CO2H)-S
er-Lys-Tyr-Leu-Glu-Aib-Lys-Ala-Ala-Gln-Asp-Phe-Val-Glu-Trp-Leu-Lys-Ala-
Gly-Gly-Pro-Ser-Ser-Gly-Ala-Pro-Pro-Pro-Ser-NH2;
 
HsQGTFTSDK(PEG2-PEG2-CO(CH2)18CO2H)SKYLE-Aib-KAAQDFVEWLK
AGGPSSGAPPPS-NH2;
Compound 3 (SEQ ID NO. 3): 
His-(D-Ser)-Gln-Gly-Thr-Phe-Thr-Ser-Asp-Lys(PEG2-PEG2-γGlu-CO(CH2)18CO
2H)-Ser-Lys-Tyr-Leu-Glu-Aib-Lys-Ala-Ala-Gln-Asp-Phe-Val-Glu-Trp-Leu-Lys-
Ala-Gly-Gly-Pro-Ser-Ser-Gly-Ala-Pro-Pro-Pro-Ser-NH2;
 
HsQGTFTSDK(PEG2-PEG2-γGlu-CO(CH2)18CO2H)SKYLE-Aib-KAAQDFVE
WLKAGGPSSGAPPPS-NH2;
Compound 4 (SEQ ID NO. 4): 
His-(D-Ser)-Gln-Gly-Thr-Phe-Thr-Ser-Asp-Lys(PEG₂-PEG₂-γGlu-CO(CH₂)₁₆CO
₂H)-Ser-Lys-Tyr-Leu-Glu-Aib-Lys-Ala-Ala-Gln-Asp-Phe-Val-Glu-Trp-Leu-Lys-
Ala-Gly-Gly-Pro-Ser-Ser-Gly-Ala-Pro-Pro-Pro-Ser-NH₂;
 
HsQGTFTSDK(PEG2-PEG2-γGlu-CO(CH2)16CO2H)SKYLE-Aib-KAAQDFVE
WLKAGGPSSGAPPPS-NH2;
Compound 5 (SEQ ID NO. 5): 
His-(D-Ser)-Gln-Gly-Thr-Phe-Thr-Ser-Asp-Lys(PEG₂-PEG₂-γGlu-CO(CH₂)₁₈CH
₃)-Ser-Lys-Tyr-Leu-Glu-Aib-Lys-Ala-Ala-Gln-Asp-Phe-Val-Glu-Trp-Leu-Lys-A
la-Gly-Gly-Pro-Ser-Ser-Gly-Ala-Pro-Pro-Pro-Ser-NH₂;
 
HsQGTFTSDK(PEG2-PEG2-γGlu-CO(CH2)18CH3)SKYLE-Aib-KAAQDFVEW
LKAGGPSSGAPPPS-NH2;
Compound 6 (SEQ ID NO. 6): 
His-(D-Ser)-Gln-Gly-Thr-Phe-Thr-Ser-Asp-Lys(PEG₂-PEG₂-γGlu-CO(CH₂)₁₈CO
₂H)-Ser-Lys-Tyr-Leu-Asp-Glu-Arg-Ala-Ala-Gln-Asp-Phe-Iva-Gln-Trp-Leu-Leu-
Asp-Gly-Gly-Pro-Ser-Ser-Gly-Aib-Pro-Pro-Pro-Ser-NH₂;
 
HsQGTFTSDK(PEG2-PEG2-γGlu-CO(CH2)18CO2H)SKYLDERAAQDF-Iva-Q
WLLDGGPSSG-Aib-PPPS-NH2 ;
Compound 7 (SEQ ID NO. 7): 
His-(D-Ser)-Gln-Gly-Thr-Phe-Thr-Ser-Asp-Lys(PEG₂-PEG₂-γGlu-CO(CH₂)₁₈CO
₂H)-Ser-Lys-Tyr-Leu-Asp-Glu-Arg-Ala-Ala-Gln-Asp-Phe-Iva-Gln-Trp-Leu-Leu-
Glu-Gly-Gly-Pro-Ser-Ser-Gly-Ala-Pro-Pro-Pro-Ser-NH₂;
 
HsQGTFTSDK(PEG2-PEG2-γGlu-CO(CH2)18CO2H)SKYLDERAAQDF-Iva-Q
WLLEGGPSSGAPPPS-NH2;
Compound 8 (SEQ ID NO. 8): 
His-Aib-Gln-Gly-Thr-Phe-Thr-Ser-Asp-Lys(PEG2-PEG2-γGlu-CO(CH2)18CO2H)
-Ser-Lys-Tyr-Leu-Asp-Glu-Arg-Ala-Ala-Gln-Asp-Phe-Iva-Gln-Trp-Leu-Leu-As
p-Gly-Gly-Pro-Ser-Ser-Gly-Aib-Pro-Pro-Pro-Ser-NH2;
 
H-Aib-QGTFTSDK(PEG2-PEG2-γGlu-CO(CH2)18CO2H)SKYLDERAAQDF-Iva
-QWLLDGGPSSG-Aib-PPPS-NH2;
Compound 9 (SEQ ID NO. 9): 
His-Aib-Gln-Gly-Thr-Phe-Thr-Ser-Asp-Lys(PEG2-PEG2-γGlu-CO(CH2)16CO2H)
-Ser-Lys-Tyr-Leu-Asp-Glu-Arg-Ala-Ala-Gln-Asp-Phe-Iva-Gln-Trp-Leu-Leu-As
p-Gly-Gly-Pro-Ser-Ser-Gly-Aib-Pro-Pro-Pro-Ser-NH2;
 
H-Aib-QGTFTSDK(PEG2-PEG2-γGlu-CO(CH2)16CO2H)SKYLDERAAQDF-Iva
-QWLLDGGPSSG-Aib-PPPS-NH2;
Compound 10 (SEQ ID NO. 10): 
His-Aib-Gln-Gly-Thr-Phe-Thr-Ser-Asp-Lys(PEG2-PEG2-γGlu-CO(CH2)18CH3)-S
er-Lys-Tyr-Leu-Asp-Glu-Arg-Ala-Ala-Gln-Asp-Phe-Iva-Gln-Trp-Leu-Leu-Asp-
Gly-Gly-Pro-Ser-Ser-Gly-Aib-Pro-Pro-Pro-Ser-NH2;
 
H-Aib-QGTFTSDK(PEG2-PEG2-γGlu-CO(CH2)18CH3)SKYLDERAAQDF-Iva-
QWLLDGGPSSG-Aib-PPPS-NH2;
Compound 11 (SEQ ID NO. 11): 
His-Aib-Gln-Gly-Thr-Phe-Thr-Ser-Asp-Lys(PEG2-PEG2-CO(CH2)18CH3)-Ser-Ly
s-Tyr-Leu-Asp-Glu-Arg-Ala-Ala-Gln-Asp-Phe-Iva-Gln-Trp-Leu-Leu-Asp-Gly-G
ly-Pro-Ser-Ser-Gly-Aib-Pro-Pro-Pro-Ser-NH2;
 
H-Aib-QGTFTSDK(PEG2-PEG2-γGlu-CO(CH2)18CH3)SKYLDERAAQDF-Iva-
QWLLDGGPSSG-Aib-PPPS-NH2;
Compound 12 (SEQ ID NO. 12): 
His-(D-Ser)-Gln-Gly-Thr-Phe-Thr-Ser-Asp-Lys(PEG2-PEG2-γGlu-CO(CH2)18CO
2H)-Ser-Lys-Tyr-Leu-Asp-Glu-Arg-Ala-Ala-Gln-Asp-Phe-Iva-Gln-Trp-Leu-Leu-
Asp-Gly-Gly-Pro-Ser-Ser-Gly-Aib-Pro-Pro-Pro-Ser-NH2;
 
HsQGTFTSDK(PEG2-PEG2-γGlu-CO(CH2)18CO2H)SKYLDERAAQDF-Iva-Q
WLLDGGPSSG-Aib-PPPS-NH2;
Compound 13 (SEQ ID NO. 13): 
His-(D-Ser)-Gln-Gly-Thr-Phe-Thr-Ser-Asp-Lys(Gly-Gly-Ser-Gly-Ser-Gly-γGlu-
CO(CH2)18-COOH)-Ser-Lys-Tyr-Leu-Glu-Aib-Lys-Ala-Ala-Gln-Asp-Phe-Val-G
lu-Trp-Leu-Lys-Ala-Gly-Gly-Pro-Ser-Ser-Gly-Ala-Pro-Pro-Pro-Ser;
 
HsQGTFTSDK(GGSGSG-γGlu-CO(CH2)18-COOH)SKYLE-Aib-KAAQDFVE
WLKAGGPSSGAPPPS;
Compound 14 (SEQ ID NO. 14): 
His-(D-Ser)-Gln-Gly-Thr-Phe-Thr-Ser-Asp-Lys(Gly-Gly-Ser-Gly-Ser-Gly-γGlu-
CO(CH2)18-COOH)-Ser-Lys-Tyr-Leu-Glu-Aib-Lys-Ala-Ala-Gln-Asp-Phe-Val-G
lu-Trp-Leu-Lys-Ala-Gly-Gly-Pro-Ser-Ser-Gly-Ala-Pro-Pro-Pro-Ser-NH2;
 
HsQGTFTSDK(GGSGSG-γGlu-CO(CH2)18-COOH)SKYLE-Aib-KAAQDFVE
WLKAGGPSSGAPPPS-NH2;
Compound 15 (SEQ ID NO. 15): 
His-Aib-Gln-Gly-Thr-Phe-Thr-Ser-Asp-Lys(Gly-Gly-Ser-Gly-Ser-Gly-γGlu-CO(
CH2)18-COOH)-Ser-Lys-Tyr-Leu-Asp-Glu-Arg-Ala-Ala-Gln-Asp-Phe-Iva-Gln-T
rp-Leu-Leu-Asp-Gly-Gly-Pro-Ser-Ser-Gly-Aib-Pro-Pro-Pro-Ser;
 
H-Aib-QGTFTSDK(GGSGSG-γGlu-CO(CH2)18-COOH)SKYLDERAAQDF-Iva
-QWLLDGGPSSG-Aib-PPPS;
Compound 16 (SEQ ID NO. 16):

His-Aib-Gln-Gly-Thr-Phe-Thr-Ser-Asp-Lys(Gly-Gly-Ser-Gly-Ser-Gly-γGlu-CO(
CH2)18-COOH)-Ser-Lys-Tyr-Leu-Asp-Glu-Arg-Ala-Ala-Gln-Asp-Phe-Iva-Gln-T
rp-Leu-Leu-Asp-Gly-Gly-Pro-Ser-Ser-Gly-Aib-Pro-Pro-Pro-Ser-NH2;

H-Aib-QGTFTSDK(GGSGSG-γGlu-CO(CH2)18-COOH)SKYLDERAAQDF-Iva
-QWLLDGGPSSG-Aib-PPPS-NH2.

In the above sequence, the modification of Lys can be one of the following structures:
Lys(PEG₂-PEG₂-γGlu-CO(CH₂)₁₈CO₂H) 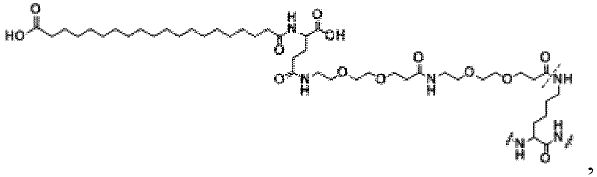 or
Lys(PEG₂-PEG₂-γGlu-CO(CH₂)₁₈CH₃) 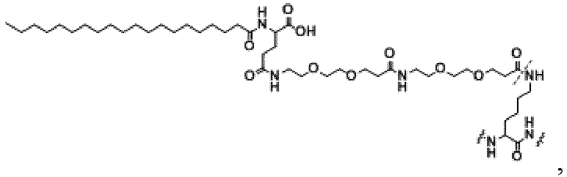 or
Lys(PEG₂-PEG₂-γGlu-CO(CH₂)₁₆CO₂H) 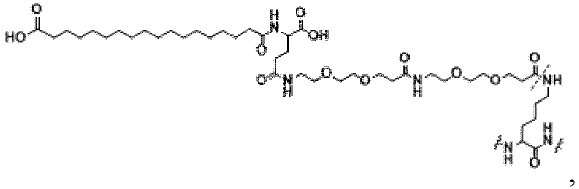 or
Lys(PEG₂-PEG₂-CO(CH₂)₁₈CO₂H) 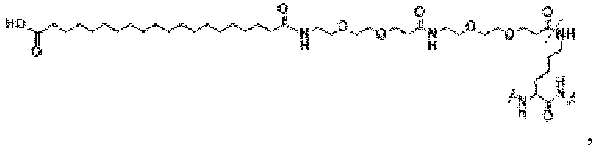 , or
Lys(Gly-Gly-Ser-Gly-Ser-Gly-γGlu-CO(CH₂)₁₈CO₂H)

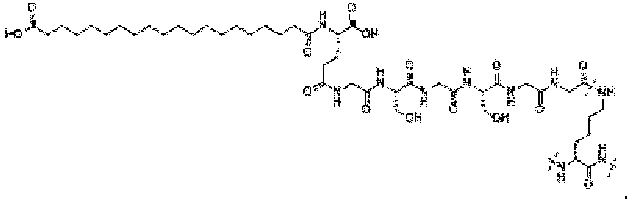

The Lys linked to the lipophilic substituent can be replaced by:

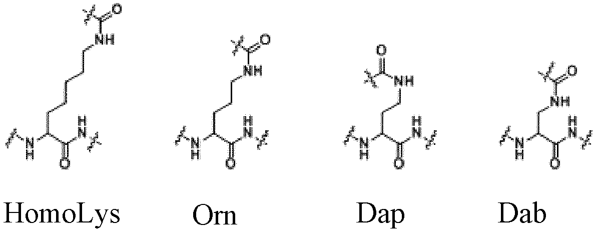

The abbreviations used in the present invention are defined as follows:
Boc is tert-butyloxycarbonyl, Fmoc is fluorenylmethoxycarbonyl, t-Bu is tert-butyl, ivDDe is 1-(4,4-dimethyl-2,6-dioxo-cyclohexylidene)-3-methyl-butyl, resin is a resin, TFA is trifluoroacetic acid, EDT is 1,2-ethanedithiol, Phenol is phenyl hydroxide, FBS is fetal bovine serum, BSA is bovine serum albumin, HPLC is high performance liquid chromatography, mPEG is mono-methoxy-polyethylene diol, His is histidine, Ser is serine, D-Ser is D-serine, Gln is glutamine, Gly is glycine, Glu is Glutamicacud, Ala is Alanine, Thr is threonine, Lys is lysine, Arg is arginine, Tyr is tyrosine, Asp is Aspartic acid, Trp is tryptophan, Phe is phenylalanine, IIe is isoleucine, Leu is Leucine, Cys is cysteine, Pro is proline, Val is Valine, Met is methionine, Asn is asparagines, HomoLys is homolysine, Orn is ornithine, Dap is diaminopimelic acid, Dab is 2,4-diaminobutyric acid, Aib is 2-aminoisobutyric acid and Iva is isovaline.

### DESCRIPTION OF THE DRAWINGS

Examples of the present invention are described in detail below with reference to the Drawings, wherein:
Fig. 1 shows the colon length (MEAN ± SEM) of each group of mice measured on the 9^{th} day of sampling in Example 2.
Fig. 2 is the daily status score bar chart of mice from Day 1 to Day 8 in Example 2.
Fig. 3 shows the HE stained slice of the mouse colon in Example 2.
Fig. 4A shows the Masson stained slice of the mouse colon in Example 2.
Fig. 4B shows the area ratio (MEAN ± SEM) of blue collagen fibers to colon tissue in Masson staining of the mouse colon in Example 2.
Fig. 5 shows the α-SMA protein immunohistochemical stained slice of mouse colon in Example 2.
Fig. 6 shows the Ly-6g protein immunohistochemical stained slice of mouse colon in Example 2.
Fig. 7 shows a bar chart of the serum cytokine (MEAN ± SEM) results in each group of mice measured on the 9^{th} day of sampling in Example 2.
Fig. 8 shows the colon length (MEAN ± SEM) of each group of mice measured at the end of three cycles in Example 3.
Fig. 9 shows the daily status score bar chart of mice during three cycles in Example 3.
Fig. 10 shows the HE stained slice of the mouse colon in Example 3.
Fig. 11A shows the Masson stained slice of the mouse colon in Example 3.
Fig. 11B shows the area ratio (MEAN ± SEM) of blue collagen fibers to colon tissue in Masson staining of the mouse colon in Example 3.
Fig. 12 shows the Fibronectin protein immunohistochemical stained slice of mouse colon in Example 3.
Fig. 13 shows the serum cytokines of each group of mice measured during sampling at the end of three cycles in Example 3 (MEAN±SEM).
Fig. 14 shows an exemplary modification of the Lys side chain in the parent peptide of the compound of the present invention.

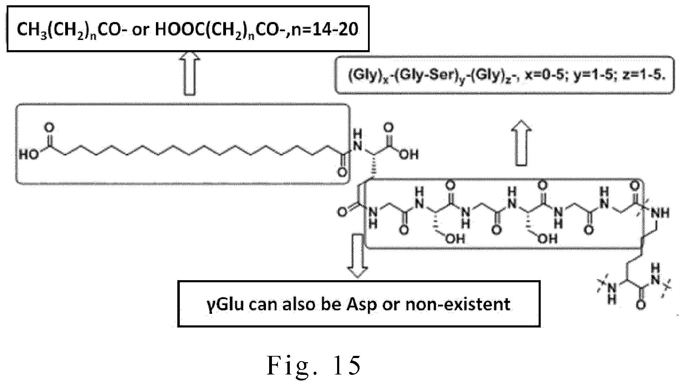

Fig. 15 shows another exemplary modification of the Lys side chain in the parent peptide of the compound of the present invention.

### DETAILED DESCRIPTION OF THE INVENTION

The examples of the present invention can be described in detail hereafter in conjunction with the examples, it will be understood by those skilled in the art will appreciate that the following examples are only intended to illustrate the invention and should not be considered as limiting the scope of the invention Where specific conditions are not indicated in the examples, they are carried out according to conventional conditions or those recommended by the manufacturer. Where the manufacturer is not specified for the reagents or apparatus used, they are conventional products available commercially.

### Example 1 Synthesis of polypeptide compound

To better describe how to prepare the polypeptide compound of the present invention, the polypeptide compound 3 in the present invention is used as an example. The steps are as follows:

### (I) Materials:

All amino acids are purchased from NovaBiochem. Unless otherwise specified, other reagents are all analytically pure and purchased from Sigma Company. CS336X polypeptide synthesizer (C S Bio of the United States). Phenomenex Luna C18 preparative column (46 mm × 250 mm) is used to purify the polypeptides. High performance liquid chromatograph is manufactured by Waters Company. MS analysis is determined by using Agilent mass spectrometer.

### (II) Method:

### 1. Synthesis of polypeptide compound 3

### Sequence:

His-(D-Ser)-Gln-Gly-Thr-Phe-Thr-Ser-Asp-Lys(PEG₂-PEG₂-γGlu-CO(CH₂)₁₈CO
₂H)-Ser-Lys-Tyr-Leu-Glu-Aib-Lys-Ala-Ala-Gln-Asp-Phe-Val-Glu-Trp-Leu-Lys-
Ala-Gly-Gly-Pro-Ser-Ser-Gly-Ala-Pro-Pro-Pro-Ser-NH₂

### 1a) Main peptide chain assembly:

The following polypeptide in a scale of 0.25mol is synthesized on a CS336X polypeptide synthesizer (CS Bio of the United States) according to Fmoc/t-Bu strategy: 
Boc-His(Boc)-D-Ser(t-Bu)-Gln(Trt)-Gly-Thr(t-Bu)-Phe-Thr(t-Bu)-Ser(tBu)-Asp(
OtBu)-Lys(PEG2-PEG2-γGlu-CO(CH2)18CO2H)-Ser(t-Bu)-Lys(Boc)-Tyr(t-Bu)-L
eu-Glu(OtBu)-Aib-Lys(Boc)-Ala-Ala-Gln(Trt)-Asp(OtBu)-Phe-Val-Glu(OtBu)-
Trp(Boc)-Leu-Lys(Boc)-Ala-Gly-Gly-Pro-Ser(t-Bu)-Ser(t-Bu)-Gly-Ala-Pro-Pro-
Pro-Ser(t-Bu)-rink amide resin
(1) Step 1: put 0.75g of Rink amide resin in dichloromethane for swelling, and rinse the resin three time by using N,N-dimethylformamide;
(2) Step 2: use Rink amide resin as the carrier and use benzotriazole-*N*,*N*,*N'*,*N'*-tetramethyluronium hexafluorophosphate, 1-hydroxybenzotriazole and *N*,*N*-diisopropylethylamine to prepare the coupling agent; use *N,N*-dimethylformamide as the solvent to start the process reaction. Complete the condensation reactions to connect Fmoc-Ser(t-Bu)-OH,Fmoc-Pro-OH (3x),Fmoc-Ala-OH,Fmoc-Gly-OH,Fmoc-Ser(t-Bu)-OH (2x),Fmoc-Pro-OH,Fmoc-Gly-OH (2x),Fmoc-Ala-OH,Fmoc-Lys(Boc)-OH,Fmoc-Leu-OH,Fmoc-Trp(Boc)-OH,Fmo c-Glu(OtBu)-OH,Fmoc-Val-OH,Fmoc-Phe-OH,Fmoc-Asp(OtBu)-OH,Fmoc-Gln (Trt)-OH,Fmoc-Ala-OH (2x),Fmoc-Lys(Boc)-OH,Fmoc-Aib-OH,Fmoc-Glu(OtBu)-OH,Fmoc-Leu-OH,F moc-Tyr(t-Bu)-OH,Fmoc-Lys(Boc)-OH,Fmoc-Ser(t-Bu)-OH,Fmoc-Lys(PEG₂-P EG₂-γGlu-CO(CH₂)₁₈CO₂H)-OH,Fmoc-Asp(OtBu)-OH,Fmoc-Ser(t-Bu)-OH,Fmo c-Thr(t-Bu)-OH,Fmoc-Phe-OH,Fmoc-Thr(t-Bu)-OH,Fmoc-Gly-OH,Fmoc-Gln(T rt)-OH,Fmoc-D-Ser(t-Bu)-OH,Boc-His(Boc)-OH,to generate
   Boc-His(Boc)-D-Ser(t-Bu)-Gln(Trt)-Gly-Thr(t-Bu)-Phe-Thr(t-Bu)-Ser(tBu )-Asp(OtBu)-Lys(PEG₂-PEG₂-γGlu-CO(CH₂)₁₈CO₂H)-Ser(t-Bu)-Lys(Boc)-Tyr(t-Bu)-Leu-Glu(OtBu)-Aib-Lys(Boc)-Ala-Ala-Gln(Trt)-Asp(OtBu)-Phe-Val-Glu(O tBu)-Trp(Boc)-Leu-Lys(Boc)-Ala-Gly-Gly-Pro-Ser(t-Bu)-Ser(t-Bu)-Gly-Ala-Pro -Pro-Pro-Ser(t-Bu)-Rink Amide resin.

### 1b) Removal of Lys(PEG₂-PEG₂-γGlu-CO(CH₂)₁₈CO₂H) and introduction of lipophilic substituent:

In the mixed solution of *N*-methylpyrrolidone and dichloromethane, rinse the protective peptidyl resin synthesized in 1a) twice, and add the fresh 2.0% hydrazine hydrate *N*-methylpyrrolidone solution to start filtration.

After that, fully rinse the resin with dichloromethane and N-methylpyrrolidone. Add the coupling solution of FmocNH-PEG₂-OH, benzotriazole-*N*,*N*,*N'*,*N'*-tetramethyluronium hexafluorophosphate, 1-hydroxybenzotriazole and diisopropylethylamine based *N*-methylpyrrolidone. Shake it for 3.0h, and complete filtration and rinsing. Repeat the hydrazine step twice. Use the piperidine/*N*,*N*-dimethylformamide solution to remove Fmoc group. Repeat the Fmoc-PEG₂-OH coupling reaction once.

Use the piperidine/ *N,N*-dimethylformamide solution to remove the Fmoc group. Then under the conventional conditions, couple Fmoc-yGlu-OtBu and tBu to generate the Icosanoic acid under single protection. Remove the polypeptide under full protection, to generate the crude compound.

His-(D-Ser)-Gln-Gly-Thr-Phe-Thr-Ser-Asp-Lys(PEG2-PEG2-γGlu-CO(CH
2)18CO2H)-Ser-Lys-Tyr-Leu-Glu-Aib-Lys-Ala-Ala-Gln-Asp-Phe-Val-Glu-Trp-Le
u-Lys-Ala-Gly-Gly-Pro-Ser-Ser-Gly-Ala-Pro-Pro-Pro-Ser-NH2

### 1c) Purification of the polypeptide compound:

The crude product from 1b) is dissolved in 5.0% acetic acid. In the acetonitrile: H₂O = 1:1 (volume ratio) solution, purify the semi-preparative HPLC twice in the 50mm × 250mm column filled with reversed C18. Use 30-60% acetonitrile-0.1% trifluoroacetic acid/H₂O gradient to elute the column for 45.0 minutes at the rate of 40 mL/min. Collect the components that comprise peptide, concentrate them to remove acetonitrile, and then lyophilize them. The pure product with HPLC purity > 95% is obtained. Use liquid chromatography-mass spectrometry to analyze the separated product. The m/z value of protonized molecular ion peak is 4860.9, and the theoretical value is 4863.5.

**Table 1. Polypeptide compound of the present invention**

| **Compound** | **Parent peptide** | **Structure** | **Theoretical value** | **Measured value** |
|---|---|---|---|---|
| 1 | SEQ ID NO. 1 | His-Aib-Gln-Gly-Thr-Phe-Thr-Ser-Asp-Lys(PEG2-PEG2-γG lu-CO(CH2)18CO2H)-Ser-Lys-Tyr-Leu-Glu-Aib-Lys-Ala-Ala -Gln-Asp-Phe-Val-Glu-Trp-Leu-Lys-Ala-Gly-Gly-Pro-Ser-S er-Gly-Ala-Pro-Pro-Pro-Ser-NH2 | 4861.5 | 4858.5 |
| 2 | SEQ ID NO. 2 | His-(D-Ser)-Gln-Gly-Thr-Phe-Thr-Ser-Asp-Lys(PEG2-PEG2 -CO(CH2)18CO2H)-Ser-Lys-Tyr-Leu-Glu-Aib-Lys-Ala-Ala- Gln-Asp-Phe-Val-Glu-Trp-Leu-Lys-Ala-Gly-Gly-Pro-Ser-Se r-Gly-Ala-Pro-Pro-Pro-Ser-NH2 | 4734.4 | 4731.5 |
| 3 | SEQ ID NO. 3 | His-(D-Ser)-Gln-Gly-Thr-Phe-Thr-Ser-Asp-Lys(PEG2-PEG2 -γGlu-CO(CH2)18CO2H)-Ser-Lys-Tyr-Leu-Glu-Aib-Lys-Ala -Ala-Gln-Asp-Phe-Val-Glu-Trp-Leu-Lys-Ala-Gly-Gly-Pro-S er-Ser-Gly-Ala-Pro-Pro-Pro-Ser-NH2 | 4863.5 | 4860.9 |
| 4 | SEQ ID NO. 4 | His-(D-Ser)-Gln-Gly-Thr-Phe-Thr-Ser-Asp-Lys(PEG₂-PEG₂ | 4835.5 | 4832.5 |
| | | -γGlu-CO(CH₂)₁₆CO₂H)-Ser-Lys-Tyr-Leu-Glu-Aib-Lys-Ala -Ala-Gln-Asp-Phe-Val-Glu-Trp-Leu-Lys-Ala-Gly-Gly-Pro-S er-Ser-Gly-Ala-Pro-Pro-Pro-Ser-NH₂ | | |
| 5 | SEQ ID NO. 5 | His-(D-Ser)-Gln-Gly-Thr-Phe-Thr-Ser-Asp-Lys(PEG2-PEG2 -γGlu-CO(CH2)18CH3)-Ser-Lys-Tyr-Leu-Glu-Aib-Lys-Ala- Ala-Gln-Asp-Phe-Val-Glu-Trp-Leu-Lys-Ala-Gly-Gly-Pro-S er-Ser-Gly-Ala-Pro-Pro-Pro-Ser-NH2 | 4833.5 | 4830.5 |
| 6 | SEQ ID NO. 6 | His-(D-Ser)-Gln-Gly-Thr-Phe-Thr-Ser-Asp-Lys(PEG₂-PEG₂ -γGlu-CO(CH₂)₁₈CO₂H)-Ser-Lys-Tyr-Leu-Asp-Glu-Arg-Ala -Ala-Gln-Asp-Phe-Iva-Gln-Trp-Leu-Leu-Asp-Gly-Gly-Pro- Ser-Ser-Gly-Aib-Pro-Pro-Pro-Ser-NH₂ | 4963.5 | 4960.5 |
| 7 | SEQ ID NO. 7 | His-(D-Ser)-Gln-Gly-Thr-Phe-Thr-Ser-Asp-Lys(PEG2-PEG2 -γGlu-CO(CH2)18CO2H)-Ser-Lys-Tyr-Leu-Asp-Glu-Arg-Ala -Ala-Gln-Asp-Phe-Iva-Gln-Trp-Leu-Leu-Glu-Gly-Gly-Pro-S er-Ser-Gly-Ala-Pro-Pro-Pro-Ser-NH2 | 4963.5 | 4960.9 |
| 8 | SEQ ID NO. 8 | His-Aib-Gln-Gly-Thr-Phe-Thr-Ser-Asp-Lys(PEG₂-PEG₂-γG lu-CO(CH₂)₁₈CO₂H)-Ser-Lys-Tyr-Leu-Asp-Glu-Arg-Ala-Al a-Gln-Asp-Phe-Iva-Gln-Trp-Leu-Leu-Asp-Gly-Gly-Pro-Ser- Ser-Gly-Aib-Pro-Pro-Pro-Ser-NH₂ | 4961.6 | 4959.3 |
| 9 | SEQ ID NO. 9 | His-Aib-Gln-Gly-Thr-Phe-Thr-Ser-Asp-Lys(PEG₂-PEG₂-γG lu-CO(CH₂)₁₆CO₂H)-Ser-Lys-Tyr-Leu-Asp-Glu-Arg-Ala-Al a-Gln-Asp-Phe-Iva-Gln-Trp-Leu-Leu-Asp-Gly-Gly-Pro-Ser- Ser-Gly-Aib-Pro-Pro-Pro-Ser-NH₂ | 4933.5 | 4930.4 |
| 10 | SEQ ID NO. 10 | His-Aib-Gln-Gly-Thr-Phe-Thr-Ser-Asp-Lys(PEG₂-PEG₂-γG lu-CO(CH₂)₁₈CH₃)-Ser-Lys-Tyr-Leu-Asp-Glu-Arg-Ala-Ala- Gln-Asp-Phe-Iva-Gln-Trp-Leu-Leu-Asp-Gly-Gly-Pro-Ser-S er-Gly-Aib-Pro-Pro-Pro-Ser-NH₂ | 4931.6 | 4928.6 |
| 11 | SEQ ID NO. 11 | His-Aib-Gln-Gly-Thr-Phe-Thr-Ser-Asp-Lys(PEG2-PEG2-CO (CH2)18CH3)-Ser-Lys-Tyr-Leu-Asp-Glu-Arg-Ala-Ala-Gln-A sp-Phe-Iva-Gln-Trp-Leu-Leu-Asp-Gly-Gly-Pro-Ser-Ser-Gly- Aib-Pro-Pro-Pro-Ser-NH2 | 4802.5 | 4799.5 |
| 12 | SEQ ID NO. 12 | His-(D-Ser)-Gln-Gly-Thr-Phe-Thr-Ser-Asp-Lys(PEG₂-PEG₂ -γGlu-CO(CH₂)₁₈CO₂H)-Ser-Lys-Tyr-Leu-Asp-Glu-Arg-Ala -Ala-Gln-Asp-Phe-Iva-Gln-Trp-Leu-Leu-Asp-Gly-Gly-Pro- Ser-Ser-Gly-Aib-Pro-Pro-Pro-Ser-NH₂ | 4963.5 | 4960.6 |
| 13 | SEQ ID NO. 13 | His-(D-Ser)-Gln-Gly-Thr-Phe-Thr-Ser-Asp-Lys(Gly-Gly-Se r-Gly-Ser-Gly-γGlu-CO(CH2)18-COOH)-Ser-Lys-Tyr-Leu-G lu-Aib-Lys-Ala-Ala-Gln-Asp-Phe-Val-Glu-Trp-Leu-Lys-Ala -Gly-Gly-Pro-Ser-Ser-Gly-Ala-Pro-Pro-Pro-Ser | 4948.5 | 4948.8 |
| 14 | SEQ ID NO. 14 | His-(D-Ser)-Gln-Gly-Thr-Phe-Thr-Ser-Asp-Lys(Gly-Gly-Se r-Gly-Ser-Gly-γGlu-CO(CH2)18-COOH)-Ser-Lys-Tyr-Leu-G lu-Aib-Lys-Ala-Ala-Gln-Asp-Phe-Val-Glu-Trp-Leu-Lys-Ala -Gly-Gly-Pro-Ser-Ser-Gly-Ala-Pro-Pro-Pro-Ser-NH2 | 4963.5 | 4962.6 |
| 15 | SEQ ID NO. 15 | His-Aib-Gln-Gly-Thr-Phe-Thr-Ser-Asp-Lys(Gly-Gly-Ser-Gl y-Ser-Gly-γGlu-CO(CH2)18-COOH)-Ser-Lys-Tyr-Leu-Asp- Glu-Arg-Ala-Ala-Gln-Asp-Phe-Iva-Gln-Trp-Leu-Leu-Asp-G ly-Gly-Pro-Ser-Ser-Gly-Aib-Pro-Pro-Pro-Ser | 5046. 4 | 5046. 8 |
| 16 | SEQ ID NO. 16 | His-Aib-Gln-Gly-Thr-Phe-Thr-Ser-Asp-Lys(Gly-Gly-Ser-Gl y-Ser-Gly-γGlu-CO(CH2)18-COOH)-Ser-Lys-Tyr-Leu-Asp- Glu-Arg-Ala-Ala-Gln-Asp-Phe-Iva-Gln-Trp-Leu-Leu-Asp-G ly-Gly-Pro-Ser-Ser-Gly-Aib-Pro-Pro-Pro-Ser-NH2 | 5061.4 | 5060.6 |

### Example 2 Preventive and therapeutic effect of polypeptide compounds 1-16 and positive control compound 6-TG on dextran sulphate sodium salt (DSS) induced acute intestinal fibrosis model of mouse

The DSS in this example is purchased from MP Biomedicals (CAS No.: 9011-18-1), and the positive control compound 6-thioguanine (6-TG) is a compound based on prior art and is purchased from Shanghai Aladdin Biochemical Technology Co., Ltd. (CAS No.: 154-42-7).

After 7 days of adaptive feeding, 114 Balb/c mice of 6-8 weeks age (purchased from Guangdong Medical Laboratory Animal Center, with weight approx. 16-20 g) are subjected to the experiment. Then randomly put the mice into 19 groups, with 6 mice in each group. The groups are normal group, model group, 6-TG group and polypeptide compound groups 1-16.

Model the 19 groups of mice and administer the drug to them for 8 days. In the 8 days, the normal group is given normal drinking water and is given subcutaneous injection of PBS once every day. From Day 1 to Day 7, the model group drinks 3% DSS and is given subcutaneous injection of PBS once every day. On Day 8, 3% DSS is replaced with normal drinking water and subcutaneous injection of PBS is given once. From Day 1 to Day 7, the 6-TG group drinks 3% DSS and is given 2 mg/kg 6-TG by the means of gavage once every day. On Day 8, 3% DSS is replaced with normal drinking water and 2 mg/kg 6-TG is given by the means of gavage once. From Day 1 to Day 7, the polypeptide compound groups 1-16 drink 3% DSS and are given subcutaneous injection of 120 µg/kg polypeptide compounds 1-16 once every day. On Day 8, 3% DSS is replaced with normal drinking water and subcutaneous injection of 120 µg/kg polypeptide compounds 1-16 is given once. Information on water drinking and drug administration of the groups of mice is shown in Table 1.

**Table 1 Water drinking and drug administration regime of the groups of mice**

| Group | Water drinking | | Drug administration regime (once/day) |
|---|---|---|---|
| | Day 1 to Day 7 | Day 8 | Day 1 to Day 8 |
| Normal group | Water | Water | PBS, s.c. |
| Model group | Water with 3% DSS | Water | PBS, s.c. |
| 6-TG group | Water with 3% DSS | Water | 2 mg/kg 6-TG, p.o. |
| Polypeptide compound group 1 | Water with 3% DSS | Water | 120 µg/kg polypeptide compound 1, s.c. |
| Polypeptide compound group 2 | Water with 3% DSS | Water | 120 µg/kg polypeptide compound 2, s.c. |
| Polypeptide compound group 3 | Water with 3% DSS | Water | 120 µg/kg polypeptide compound 3, s.c. |
| Polypeptide compound group 4 | Water with 3% DSS | Water | 120 µg/kg polypeptide compound 4, s.c. |
| Polypeptide compound group 5 | Water with 3% DSS | Water | 120 µg/kg polypeptide compound 5, s.c. |
| Polypeptide compound group 6 | Water with 3% DSS | Water | 120 µg/kg polypeptide compound 6, s.c. |
| Polypeptide compound group 7 | Water with 3% DSS | Water | 120 µg/kg polypeptide compound 7, s.c. |
| Polypeptide compound group 8 | Water with 3% DSS | Water | 120 µg/kg polypeptide compound 8, s.c. |
| Polypeptide compound group 9 | Water with 3% DSS | Water | 120 µg/kg polypeptide compound 9, s.c. |
| Polypeptide compound group 10 | Water with 3% DSS | Water | 120 µg/kg polypeptide compound 10, s.c. |
| Polypeptide compound group 11 | Water with 3% DSS | Water | 120 µg/kg polypeptide compound 11, s.c. |
| Polypeptide compound group 12 | Water with 3% DS S | Water | 120 µg/kg polypeptide compound 12, s.c. |
| Polypeptide compound group 13 | Water with 3% DSS | Water | 120 µg/kg polypeptide compound 13, s.c. |
| Polypeptide compound group 14 | Water with 3% DSS | Water | 120 µg/kg polypeptide compound 14, s.c. |
| Polypeptide compound group 15 | Water with 3% DSS | Water | 120 µg/kg polypeptide compound 15, s.c. |
| Polypeptide compound group 16 | Water with 3% DSS | Water | 120 µg/kg polypeptide compound 16, s.c. |

| | | | |
|---|---|---|---|
| Note: s.c.: subcutaneous injection; p.o.: gavage. | | | |

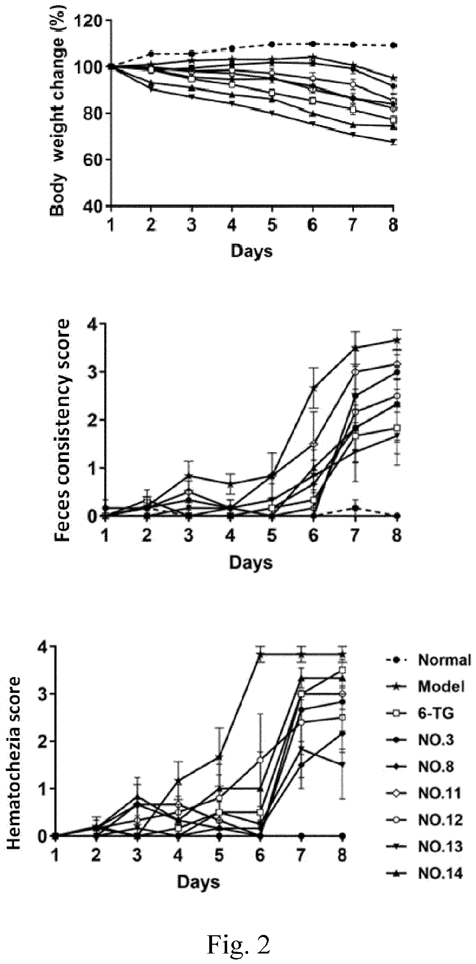

In the drug administration period, the randomly selected polypeptide compound groups 3, 8, 11, 12, 13 and 14 and the normal group, model group and 6-TG group are used for testing, and the body weight, feces consistency and feces OB of the mice are recorded every day. The GraphPad Prism 8.3.0 software is used to plot the data into a diagram. The results are shown in Fig. 2. Feces OB is measured by the benzidine method. The scoring methods for feces consistency and feces OB are given in Table 2:

**Table 2 Feces scoring methods**

| Score | Fecal property | Hematochezia |
|---|---|---|
| 0 | Normal | Occult blood (-) |
| 1 | Soft feces | Occult blood (+) light blue |
| 2 | Feces clearly wet and soft | Occult blood (+) dark blue |
| 3 | (Anus wet) semi-loose feces | Visible bloody feces |
| 4 | (Anus wet) loose feces | Visible bloody feces |

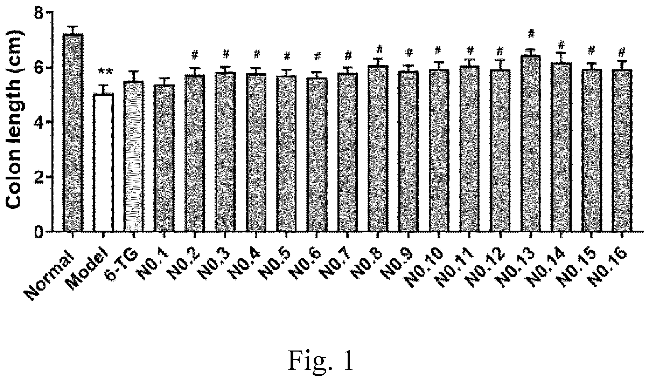

On the 9^{th} day, all mice are killed. The serums and colon tissues of the mice are collected. The colon and cecum of the mice are removed together. The colon length is measured. The GraphPad Prism 8.3.0 software is used to plot the data into a diagram. The results are shown in Fig. 1.

The serums of the mice from the randomly selected polypeptide compound groups 3, 8, 11, 12, 13 and 14 of the present invention and the normal group and model group are used for cytokine test. The results are shown in Fig. 7.

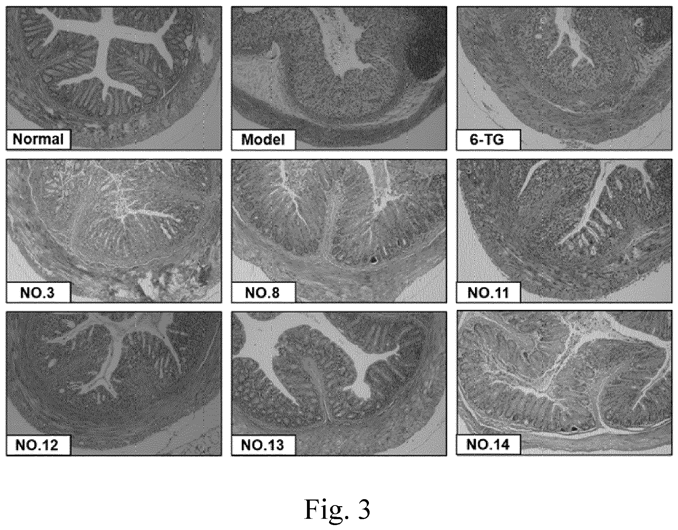

The colons of the mice from the randomly selected polypeptide compound groups 3, 8, 11, 12, 13 and 14 and the normal group, model group and 6-TG group in the present invention are taken. Approx. 5 mm of intestinal segment is taken from the distal end of each colon and is fixed in 10% formalin. The specimens are fixed, dehydrated, wrapped in paraffin and sliced (5 µm). Then tissue HE staining, Masson staining, α-SMA α-SMA protein immunohistochemical staining and Ly-6g protein immunohistochemical staining are completed. The results are shown in Fig. 3~6.

The remaining tissues are stored in -80°C freezer to be used in the subsequent index tests.

### (1) The effect of the polypeptide compounds of the present invention on the colon length in the DSS induced acute intestinal fibrosis model

Fig. 1 shows the colon length (MEAN ± SEM) of each group of mice measured during sampling on the 9^{th} day in this example. Compared with the normal group, ^{**}*P*≤0.01; Compared with the model group, ^{#}*P*≤0.05. Compared with the mice of normal group, the colon length of the mice of the model group is apparently shorter (^{**}*P*≤0.01). Compared with the mice of the model group, administration of the polypeptide compounds 1-16 of the present invention results in apparent increase of colon length of mice (^{#}*P*≤0.05). This indicates that the polypeptide compound of the present invention apparently increases the colon length of the mice who suffer DSS induced intestinal fibrosis.

### (2) The effect of the polypeptide compounds of the present invention on the daily conditions of mouse in the DSS induced acute intestinal fibrosis model

Fig. 2 shows the scoring histogram for the daily conditions of mice on Day 1 to Day 8 in this example. The daily conditions scores include body weight, feces consistency and feces OB scores (MEAN±SEM). According to daily observation, the mice of the model group have loose feces attached to their anuses, visible hematochezia and gradual weight loss. The feces consistency and feces OB scores are constantly high. Compared with the mice of model group, as indicated in Fig. 2, daily administration of 6-TG through gavage and subcutaneous injection of the polypeptide compound in the present invention will decrease the body weight of the mice who suffer DSS induced intestinal fibrosis and noticeably improve the loose feces and hematochezia conditions of the mice who suffer intestinal fibrosis.

### (3) The effect of the polypeptide compounds of the present invention on the tissue morphology of mouse in the DSS induced acute intestinal fibrosis model

Fig. 3 shows the HE stained slice of the mouse colon in this example. The microscopic magnification in the slice is 200×. As indicated in Fig. 3, the mice of normal group have intact colonic mucosa structure and well-arranged goblet cells, and no inflammatory cell infiltration is observed at the mucosa. Compared with the normal group, the colon tissues of the mice of model group are disordered, the mucous layer is damaged and the goblet cells are missing. Compared with the model group, the colon tissues of the mice of 6-TG group are relatively improved. Compared with the model group, administration of the randomly selected polypeptide compounds 3, 8, 11, 12, 13 and 14 of the present invention allows the colon structure, goblet cells and mucous layer structure of the mice to become intact. Compared with 6-TG group, administration of the polypeptide compound in the present invention allows the colon structure of the mice to become more intact. As has been proven, the polypeptide compound in the present invention will obviously improve the colon structure disorder condition of the mice in the DSS induced acute intestinal fibrosis model, and the polypeptide compound in the present invention is more efficacious in treatment of acute intestinal fibrosis model.

### (4) The effect of the polypeptide compounds of the present invention on the intestinal fibrosis of mouse in the DSS induced acute intestinal fibrosis model

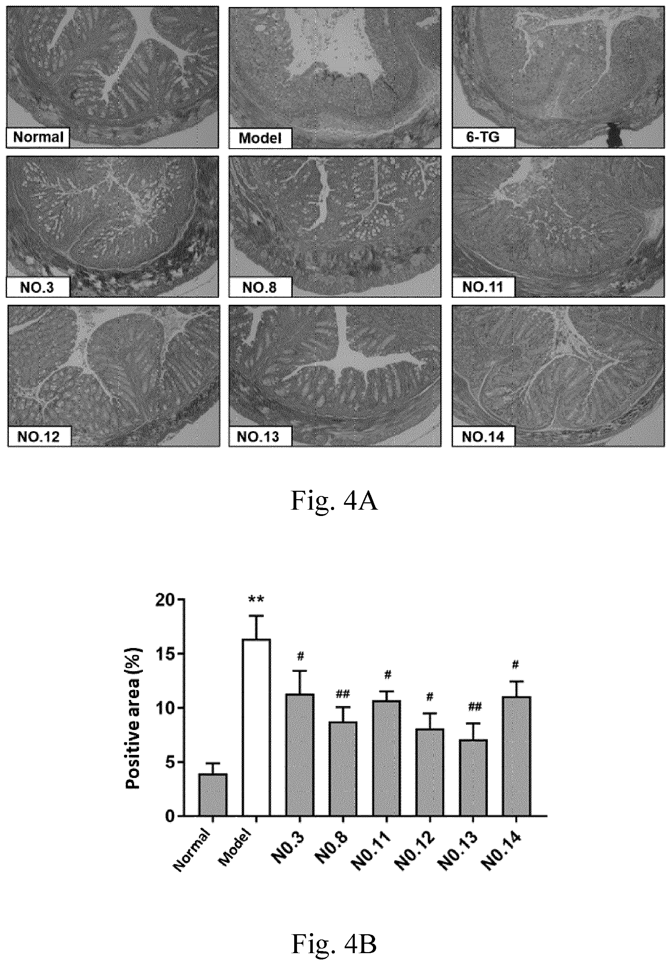

Fig. 4A shows the mouse colon Masson stained slice in this example. The microscopic magnification in the slice is 200×. Compared with the normal group, as indicated in Fig. 4A, the colon tissues of the mice of model group have higher level of Masson blue staining and the collagenous fiber is clearly increased. Compared with the model group, administration of the polypeptide compounds 3, 8, 11, 12, 13 and 14 of the present invention allows collagenous fiber of colon tissues of the mice to noticeably decrease. This indicates that the polypeptide compound in the present invention will obviously improve the intestinal fibrosis in the DSS induced acute model.

The level of blue staining of tissue is semi-quantified through Image-Pro-Plus. Fig. 4B shows the area ratio (MEAN ± SEM) of blue collagen fibers to colon tissue in Masson staining of the mouse colon in this example. Compared with normal group, **P*≤0.05; Compared with model group, ^{#}*P*≤0.05. Compared with the model group, administration of the polypeptide compounds 3, 8, 11, 12, 13 and 14 of the present invention allows the blue staining area of colon tissues of the mice to noticeably decrease. The fibrosis of colon tissues of the mice of 6-TG is not improved. This indicates that 6-TG has no therapeutic effect on intestinal fibrosis, and the polypeptide compound in the present invention has noticeable therapeutic effect on intestinal fibrosis in the acute model.

### (5) The effect of the polypeptide compounds of the present invention on the mouse colon α-SMA protein expression in the DSS induced acute intestinal fibrosis model

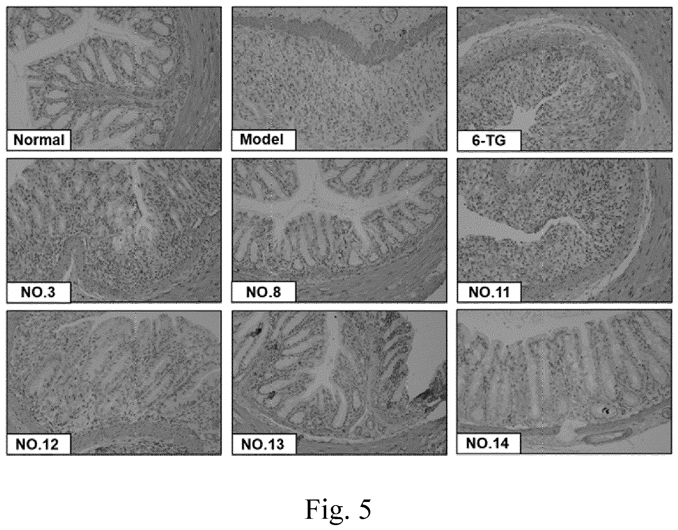

Fig. 5 shows the mouse colon α-SMA protein immunohistochemical stained slice in this example. The microscopic magnification in the slice is 400×. The outer layer of colon is muscular layer, the inner layer is mucous layer, and α-SMA is smooth muscle actin. As indicated in Fig. 5, the outermost muscular layer of the mouse colon in each group shows positive staining, but the mucous layer α-SMA protein expression differs. The normal group has low level of mucous layer α-SMA protein expression, and the model group has clearly increased α-SMA. After administration of the polypeptide compounds 3, 8, 11, 12, 13 and 14, the level of α-SMA protein expression is clearly decreased. This indicates that the polypeptide compound in the present invention can obviously decrease the level of α-SMA protein expression and inhibit intestinal fibrosis of mouse in the acute model.

### (6) The effect of the polypeptide compounds of the present invention on the mouse colon Ly-6g protein expression in the DSS induced acute intestinal fibrosis model

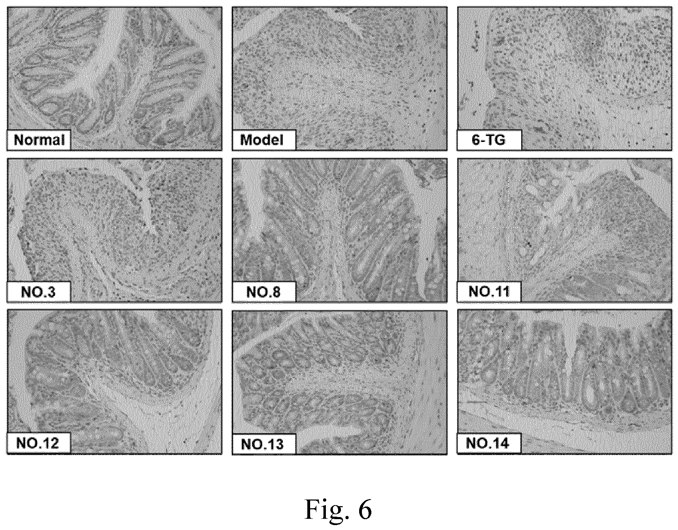

Fig. 6 shows the Ly-6g protein immunohistochemical stained slice of mouse colon in this example. The microscopic magnification is 400×. Ly-6g is the marker protein of neutrophile granulocyte. Compared with the normal group, as indicated in Fig. 6, the model group has large number of Ly-6g⁺ cells, which indicates that the mouse colon tissues have high level of neutrophile granulocyte infiltration. After administration of the polypeptide compounds 3, 8, 11, 12, 13 and 14, the Ly-6g⁺ cells clearly decrease. This indicates that the polypeptide compound of the present invention can inhibit the neutrophile granulocyte infiltration in the acute intestinal fibrosis model.

### (7) How the polypeptide compound in the present invention will affect the serum cytokines content of mouse in the DSS induced acute intestinal fibrosis model

Fig. 7 shows the statistical diagram for serum cytokines content of mouse in this example (MEAN±SEM). Compared with the normal group, ^{*}*P*≤0.05, ^{**}*P*≤0.01; Compared with model group, ##*P*≤0.01. Compared with the normal group, as indicated in Fig. 7, the content of serum proinflammatory factor IL-6 and fibrosing factor MMP-12 in the model group clearly increases, which indicates that the inflammation and level of fibrosis in the mouse body increase. After administration of the polypeptide compounds 3, 8, 11, 12, 13 and 14, the content of serum IL-6 and MMP-12 clearly decreases. This indicates that the polypeptide compound in the present invention can alleviate the inflammation and level of fibrosis of mouse in the acute intestinal fibrosis model.

### Example 3 Preventive and therapeutic effect of polypeptide compounds 1-16 on DSS induced acute intestinal fibrosis model of mouse

After 7 days of adaptive feeding, 114 Balb/c mice of 6-8 weeks age (purchased from Guangdong Medical Laboratory Animal Center, with weight approx. 16-20 g) are subjected to the experiment. Then randomly put the mice into 19 groups, with 6 mice in each group. The groups are normal group, model group, 6-TG group and polypeptide compound groups 1-16.

In the whole process, the normal group is given normal drinking water and is given subcutaneous injection PBS once every day. The model group is first given 2% DSS for 5 days and then given normal drinking water for 2 weeks. This is 1 cycle. The cycle is done 3 times. Subcutaneous injection of PBS is given once every day. The 6-TG group is first given 2% DSS for 5 days and then given normal drinking water for 2 weeks. This is 1 cycle. The cycle is done 3 times. 2 mg/kg 6-TG is given by the means of gavage once every day. The polypeptide compound groups 1-16 are first given 2% DSS for 5 days and then given normal drinking water for 2 weeks. This is 1 cycle. The cycle is done 3 times. Subcutaneous injection of 120 µg/kg polypeptide compounds 1-16 is given once every day. Water drinking and drug administration of the groups of mice are given in Table 2.

**Table 2 Water drinking and drug administration regime of the groups of mice**

| Group | Single cycle | | Drug administration regime (once/day) |
|---|---|---|---|
| | Day 1-5 | Day 6-19 | q.d. |
| Normal group | Water | Water | PBS, s.c. |
| Model group | Water with 2% DSS | Water | PBS, s.c. |
| 6-TG group | Water with 2% DSS | Water | 2 mg/kg 6-TG, p.o. |
| Polypeptide compound group 1 | Water with 2% DSS | Water | 120 µg/kg polypeptide compound 1, s.c. |
| Polypeptide compound group 2 | Water with 2% DSS | Water | 120 µg/kg polypeptide compound 2, s.c. |
| Polypeptide compound group 3 | Water with 2% DSS | Water | 120 µg/kg polypeptide compound 3, s.c. |
| Polypeptide compound group 4 | Water with 2% DSS | Water | 120 µg/kg polypeptide compound 4, s.c. |
| Polypeptide compound group 5 | Water with 2% DSS | Water | 120 µg/kg polypeptide compound 5, s.c. |
| Polypeptide compound group 6 | Water with 2% DSS | Water | 120 µg/kg polypeptide compound 6, s.c. |
| Polypeptide compound group 7 | Water with 2% DSS | Water | 120 µgkg polypeptide compound 7, s.c. |
| Polypeptide compound group 8 | Water with 2% DSS | Water | 120 µg/kg polypeptide compound 8, s.c. |
| Polypeptide compound group 9 | Water with 2% DSS | Water | 120 µg/kg polypeptide compound 9, s.c. |
| Polypeptide compound group 10 | Water with 2% DSS | Water | 120 µg/kg polypeptide compound 10, s.c. |
| Polypeptide compound group 11 | Water with 2% DSS | Water | 120 µg/kg polypeptide compound 11, s.c. |
| Polypeptide compound group 12 | Water with 2% DSS | Water | 120 µg/kg polypeptide compound 12, s.c. |
| Polypeptide compound group 13 | Water with 2% DSS | Water | 120 µg/kg polypeptide compound 13, s.c. |
| Polypeptide compound group 14 | Water with 2% DSS | Water | 120 µg/kg polypeptide compound 14, s.c. |
| Polypeptide compound group 15 | Water with 2% DSS | Water | 120 µg/kg polypeptide compound 15, s.c. |
| Polypeptide compound group 16 | Water with 2% DSS | Water | 120 µg/kg polypeptide compound 16, s.c. |

| | | | |
|---|---|---|---|
| Note: q.d.: once every day; s.c.: subcutaneous injection; p.o.: gavage. | | | |

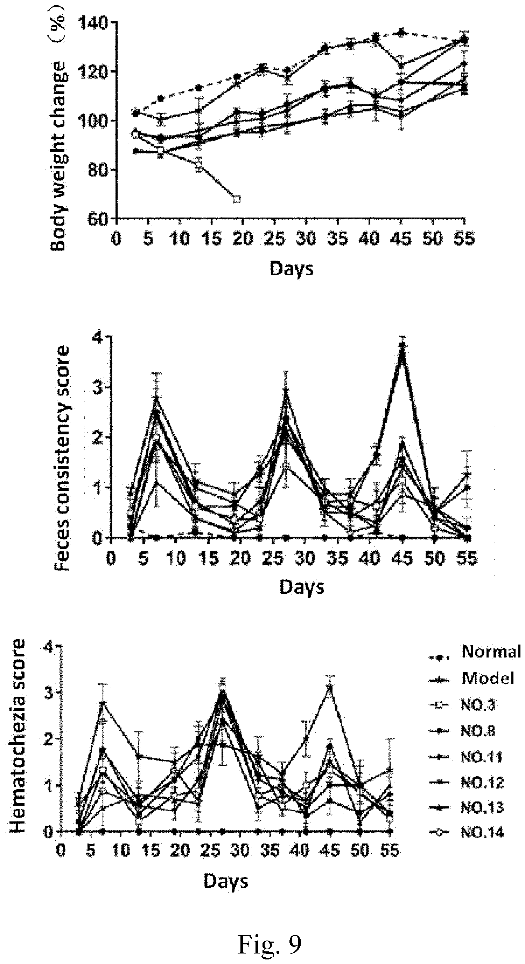

During the administration period, the polypeptide compound groups 3, 8, 11, 12, 13 and 14 and the normal group, model group and 6-TG group are randomly selected, and the body weight, feces consistency and feces OB of the mice are recorded every day. The GraphPad Prism 8.3.0 software is used to plot the data into a diagram. The results are shown in Fig. 9.

After completion of the 3rd cycle, the mice are dissected. The serums and colon tissues of the mice are collected. The colon and cecum are removed together. The colon length is measured. The GraphPad Prism 8.3.0 software is used to plot the data into a diagram. The results are shown in Fig. 8.

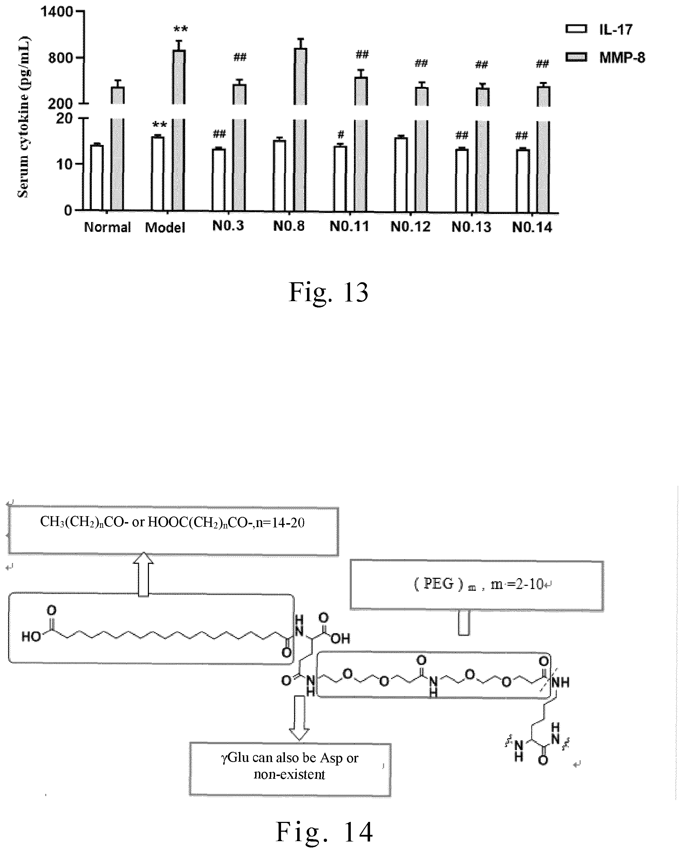

The serums of the mice from the randomly selected polypeptide compound groups 3, 8, 11, 12, 13 and 14 and the normal group and model group of the present invention are used for cytokine test. The results are shown in Fig. 13.

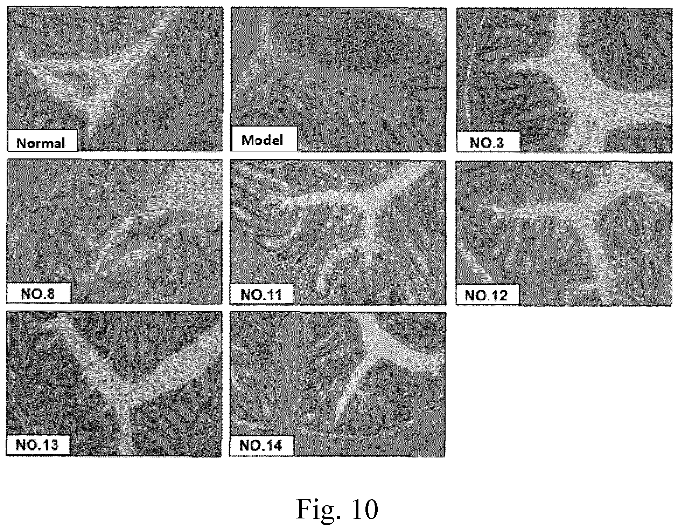

The colons of the mice from the randomly selected polypeptide compound groups 3, 8, 11, 12, 13 and 14 and the normal group and model group in the present invention are taken. Approx. 5 mm of intestinal segment is taken from the distal end of each colon and is fixed in 10% formalin. The specimens are fixed, dehydrated, wrapped in paraffin and sliced (5 µm). Then tissue HE staining, Masson staining and Fibronectin protein immunohistochemical staining are completed. The results are shown in Fig. 10~12.

The remaining tissues are stored in -80°C freezer to be used in the subsequent index tests.

### (1) The effect of the polypeptide compounds of the present invention on the mouse colon length in the DSS induced chronic intestinal fibrosis model

Fig. 8 shows the colon length (MEAN ± SEM) of each group of mice measured during sampling in this example. Compared with normal group, *P<0.05. Compared with can the mice of normal group, the colon length of the mice of model group is apparently shorter (**P*≤0.05). Compared with the mice of model group, administration of the polypeptide compounds 1-16 in the present invention results in no noticeable change of colon length of mice

### (2) The effect of the polypeptide compounds of the present invention on the daily conditions of mouse in the DSS induced chronic intestinal fibrosis model

Fig. 9 shows the scoring histogram for the daily conditions in the three cycles in this example. The daily conditions scores include body weight, feces consistency and feces OB scores (MEAN±SEM). Compared with the mice of model group, as indicated in Fig. 9, daily subcutaneous injection of the polypeptide compound in the present invention can decrease the body weight of the mice in the DSS induced chronic intestinal fibrosis model and noticeably improve the loose feces and hematochezia conditions of the mice in the chronic intestinal fibrosis model.

### (3) The effect of the polypeptide compounds of the present invention on the tissue morphology of mouse in the DSS induced chronic intestinal fibrosis model

Fig. 10 shows the HE stained slice of the mouse colon in this example. The microscopic magnification in the slice is 400×. As indicated in Fig. 10, the mice of normal group have intact colonic mucosa structure and well-arranged goblet cells, and no inflammatory cell infiltration is observed at the mucosa. Compared with the normal group, the colon tissues of the mice of model group are disordered, the mucous layer is damaged and the goblet cells are missing. Compared with the model group, administration of the randomly selected polypeptide compounds 3, 8, 11, 12, 13 and 14 in the present invention allows the colon structure, goblet cells and mucous layer structure of the mice to become intact. As has been proven, the polypeptide compound in the present invention can obviously improve the colon structure disorder condition of the mice in the DSS induced chronic intestinal fibrosis model, and the polypeptide compound in the present invention is more efficacious in treatment of chronic intestinal fibrosis model.

### (4) The effect of the polypeptide compounds of the present invention on the intestinal fibrosis of mouse in the DSS induced chronic intestinal fibrosis model

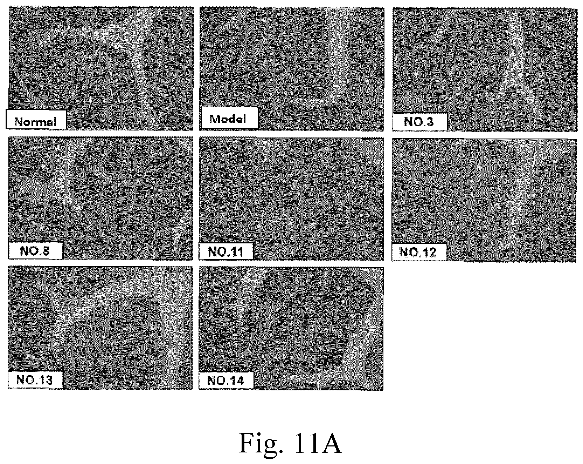

Fig. 11A shows the mouse colon Masson stained slice in this example. The microscopic magnification in the slice is 400×. Compared with the normal group, as indicated in Fig. 11A, the colon tissues of the mice of model group have higher level of Masson blue staining and the collagenous fiber is clearly increased. Compared with the model group, administration of the polypeptide compounds 3, 8, 11, 12, 13 and 14 in the present invention allows collagenous fiber of colon tissues of the mice to noticeably decrease. This indicates that the polypeptide compound in the present invention can obviously improve the intestinal fibrosis in the DSS induced chronic model.

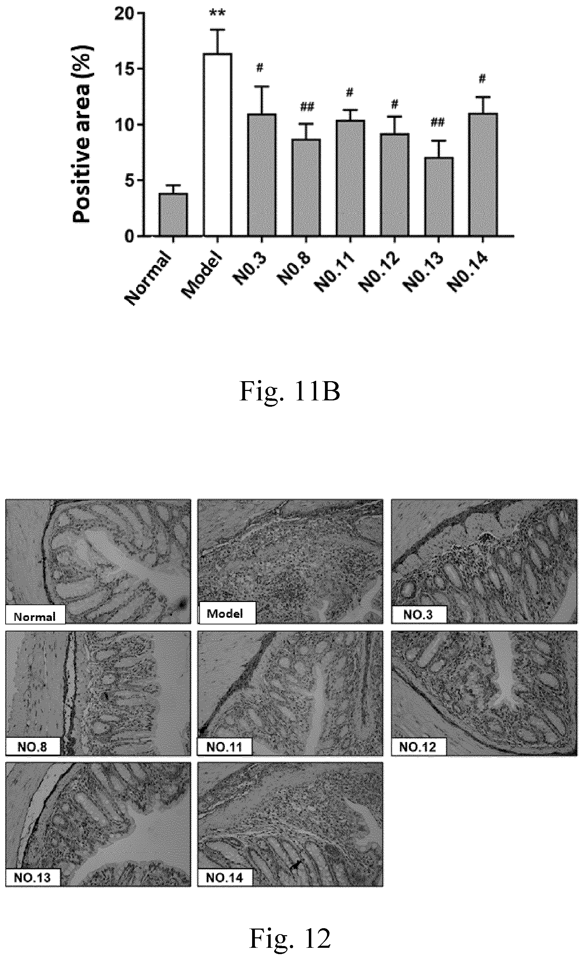

The level of blue staining of tissue is semi-quantified through Image-Pro-Plus. Fig. 11B shows the area ratio (MEAN ± SEM) of blue collagen fibers to colon tissue in Masson staining of the mouse colon in this example. Compared with normal group, ****P*≤0.01; Compared with the model group, ^{#}*P*≤0.05, ##*P*≤0.01. Compared with the model group, administration of the polypeptide compounds 3, 8, 11, 12, 13 and 14 in the present invention allows the blue staining area of colon tissues of the mice to noticeably decrease. This indicates that the polypeptide compound in the present invention has noticeable therapeutic effect on intestinal fibrosis.

### (5) The effect of the polypeptide compounds of the present invention on the mouse colon Fibronectin protein expression in the DSS induced chronic intestinal fibrosis model

Fig. 12 shows the Fibronectin protein immunohistochemical stained slice of mouse colon in this example. The microscopic magnification in the slice is 400×. As indicated in Fig. 12, the submucous layer of mouse colon in each group has Fibronectin deposition, but the level of mucous layer Fibronectin protein expression differs. The normal group has low level of mucous layer Fibronectin protein expression, and the model group has clearly increased Fibronectin. After administration of the polypeptide compounds 3, 8, 11, 12, 13 and 14, the level of Fibronectin protein expression is clearly decreased. This indicates that the polypeptide compound in the present invention can obviously decrease the level of Fibronectin protein expression and inhibit intestinal fibrosis of mouse.

### (6) The effect of the polypeptide compounds of the present invention on the serum cytokines content of mouse in the DSS induced chronic intestinal fibrosis model

Fig. 13 shows the statistical diagram for serum cytokines content of mouse in this example (MEAN±SEM). Compared with normal group, ****P*≤0.01; Compared with the model group, ^{#}*P*≤0.05, ^{##}*P*≤0.01. Compared with the normal group, as indicated in Fig. 13, the content of serum proinflammatory factor IL-6 and fibrosing factor MMP-8 in the model group clearly increases, which indicates that the inflammation and level of fibrosis in the mouse body increase. After administration of the polypeptide compounds 3, 8, 11, 12, 13 and 14, the content of serum IL-6 and MMP-8 clearly decreases. This indicates that the polypeptide compound in the present invention can alleviate the inflammation and level of fibrosis of mouse in the chronic model.

In summary, after further optimization of amino acid sequence and modification of amino acid side chain, the polypeptide compound in the present invention can decrease the serum proinflammatory factor, inhibit neutrophile granulocyte infiltration and alleviate the inflammation. It can also decrease the fibrosing factor, lower the level of α-SMA and Fibronectin protein expression, and have noticeable therapeutic effect on intestinal fibrosis. It can also control the body weight of mouse. It is more advantageous in clinical use against Crohn's disease, colonitis, fibrosis induced by Crohn's disease and fibrosis induced colonitis. In terms of therapeutic effect, the polypeptide compound in the present invention is superior to the 6-thioguanine (6-TG) that is currently used clinically.

It is understood that the present invention is not intended to be limited to the above detailed description of the preferred examples. All modifications and deformations made by those skilled in the art without departing from the spirit of the present invention can be incorporated in the protection scope of the appended claims of the present invention.

## Claims

1. Use of a polypeptide compound in the preparation of drugs for preventing or treating inflammatory bowel diseases and related intestinal fibrosis, wherein the polypeptide compound comprises a parent peptide represented by the following amino acid sequence: 
His-Xaa2-Gln-Gly-Thr5-Phe-Thr-Ser-Asp-Lys10-Ser-Lys-Tyr-Leu-Xaa1515-
Xaa16-Xaa17-Ala-Ala-Gln20-Xaa21-Phe-Xaa23-Xaa24-Trp25-Leu-Xaa27-Xaa28-
Gly-Gly30-Pro-Ser-Ser-Gly-Xaa3535-Pro-Pro-Pro-Ser,
 wherein,
Xaa2 = Aib, Ser or D-Ser;
Xaa15 = Asp or Glu;
Xaa16 = Aib or Glu;
Xaa17 = Lys or Arg;
Xaa21 = Asp or Glu;
Xaa23 = Val or Iva;
Xaa24 = Glu or Gin;
Xaa27 = Leu or Lys;
Xaa28 = Asp, Glu or Ala;
Xaa35 = Ala or Aib.

2. The use according to Claim 1, wherein Xaa2=Aib or D-Ser.

3. The use according to Claim 2, wherein Xaa21 = Asp.

4. The use according to Claim 3, wherein the carboxyl terminal of the amino acid sequence of the parent peptide is unmodified or amino-modified to form a -CONH₂ group.

5. The use according to any one of Claims 1-4, wherein the side chain of Lys at position 10 or 12 in the amino acid sequence of the parent peptide is linked to a lipophilic substituent via a bridging group; the bridging group is one of (PEG)ₘ, (PEG)ₘ-γGlu, (PEG)ₘ-Asp, (Gly)ₓ-(Gly-Ser)_{y}-(Gly)_{z}-, (Gly)ₓ-(Gly-Ser)_{y}-(Gly)_{z}-γGlu and (Gly)ₓ-(Gly-Ser)_{y}-(Gly)_{z}-Asp; the linkage is that the side-chain amino group of Lys at position 10 or 12 forms an amide bond with the carboxyl group of the glycine residue or the (PEG)ₘ-terminal-modified carboxyl group at a terminal of the bridging group and the lipophilic substituent is connected by forming an amide bond between its carboxyl group and the amino group of the bridging group at the other terminal; the lipophilic substituent is CH₃(CH₂)ₙC(O)- or HOOC(CH₂)ₙC(O)- and its acyl group forms an amide bond with the amino group in the bridging group; wherein m is an integer from 2 to 10, n is an integer from 14-20; x is an integer from 0 to 5; y is an integer from 1 to 5; z is an integer from 1 to 5.

6. The use according to Claim 1, wherein the Lys at position 10 or 12 in the amino acid sequence of the parent peptide is replaced by HomoLys, Orn, Dap or Dab.

7. The use according to Claim 1, wherein in the amino acid sequence of the parent peptide:
Xaa2 = Aib or D-Ser;
Xaa15 = Glu;
Xaa16 = Aib;
Xaa17 = Lys;
Xaa21 = Asp;
Xaa23 = Val;
Xaa24 = Glu;
Xaa27 = Lys;
Xaa28 = Ala;
Xaa35 = Ala.

8. The use according to Claim 1, wherein in the amino acid sequence of the parent peptide:
Xaa2 = Aib or D-Ser;
Xaa15 = Asp;
Xaa16 = Glu;
Xaa17 = Arg;
Xaa21 = Asp;
Xaa23 = Iva;
Xaa24 = Gin;
Xaa27 =Leu;
Xaa28 = Asp or Glu;
Xaa35 = Ala or Aib.

9. The use according to Claim 1, wherein the amino acid sequence of the parent peptide is selected from SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15 and SEQ ID NO. 16.

10. The use according to Claim 5, wherein Lys at position 10 or 12 in the amino acid sequence of the parent peptide is linked to the lipophilic substituent via the bridging group to form the following structure:
Lys(PEG₂-PEG₂-γGlu-CO(CH₂)₁₈CO₂H) 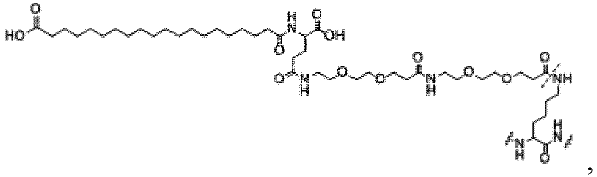 or
Lys(PEG₂-PEG₂-γGlu-CO(CH₂)₁₈CH₃) 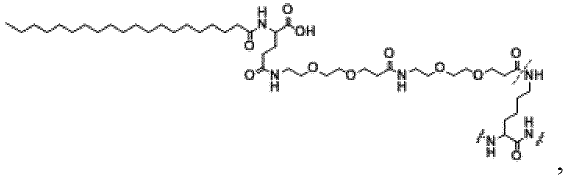 or
Lys(PEG₂-PEG₂-γGlu-CO(CH₂)₁₆CO₂H) 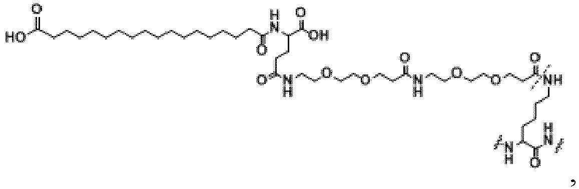 or
Lys(PEG₂-PEG₂-CO(CH₂)₁₈CO₂H) 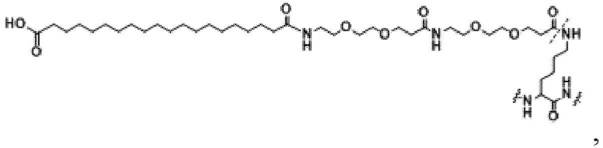 or
Lys(Gly-Gly-Ser-Gly-Ser-Gly-γGlu-CO(CH₂)₁₈CO₂H)

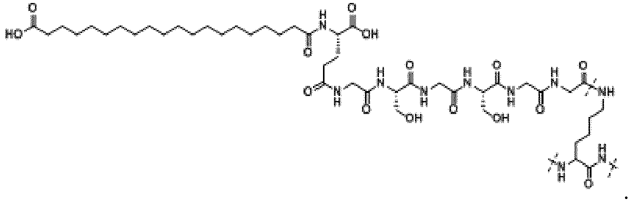

11. The use according to Claim 1, wherein the polypeptide compound is one of the following compounds:
Compound 1: 
H-Aib-QGTFTSDK(PEG2-PEG2-γGlu-CO(CH2)18CO2H)SKYLE-Aib-KAA
QDFVEWLKAGGPSSGAPPPS-NH2
Compound 2: 
HsQGTFTSDK(PEG2-PEG2-CO(CH2)18CO2H)SKYLE-Aib-KAAQDFVEW
LKAGGPSSGAPPPS-NH2
Compound 3: 
HsQGTFTSDK(PEG2-PEG2-γGlu-CO(CH2)18CO2H)SKYLE-Aib-KAAQDF
VEWLKAGGPSSGAPPPS-NH2
Compound 4: 
HsQGTFTSDK(PEG2-PEG2-γGlu-CO(CH2)16CO2H)SKYLE-Aib-KAAQDF
VEWLKAGGPSSGAPPPS-NH2
Compound 5: 
HsQGTFTSDK(PEG2-PEG2-γGlu-CO(CH2)18CH3)SKYLE-Aib-KAAQDFV
EWLKAGGPSSGAPPPS-NH2
Compound 6: 
HsQGTFTSDK(PEG2-PEG2-γGlu-CO(CH2)18CO2H)SKYLDERAAQDF-Iv
a-QWLLDGGPSSG-Aib-PPPS-NH2
Compound 7: 
HsQGTFTSDK(PEG2-PEG2-γGlu-CO(CH2)18CO2H)SKYLDERAAQDF-Iv
a-QWLLEGGPSSGAPPPS-NH2
Compound 8: 
H-Aib-QGTFTSDK(PEG2-PEG2-γGlu-CO(CH2)18CO2H)SKYLDERAAQD
F-Iva-QWLLDGGPSSG-Aib-PPPS-NH2
Compound 9: 
H-Aib-QGTFTSDK(PEG₂-PEG₂-γGlu-CO(CH₂)₁₆CO₂H)SKYLDERAAQD
F-Iva-QWLLDGGPSSG-Aib-PPPS-NH₂
Compound 10: 
H-Aib-QGTFTSDK(PEG2-PEG2-γGlu-CO(CH2)18CH3)SKYLDERAAQDF-
Iva-QWLLDGGPSSG-Aib-PPPS-NH2
Compound 11: 
H-Aib-QGTFTSDK(PEG2-PEG2-γGlu-CO(CH2)18CH3)SKYLDERAAQDF-
Iva-QWLLDGGPSSG-Aib-PPPS-NH2
Compound 12: 
HsQGTFTSDK(PEG2-PEG2-γGlu-CO(CH2)18CO2H)SKYLDERAAQDF-Iv
a-QWLLDGGPSSG-Aib-PPPS-NH2
Compound 13: 
HsQGTFTSDK(GGSGSG-γGlu-CO(CH2)18-COOH)SKYLE-Aib-KAAQDF
VEWLKAGGPSSGAPPPS
Compound 14: 
HsQGTFTSDK(GGSGSG-γGlu-CO(CH2)18-COOH)SKYLE-Aib-KAAQDF
VEWLKAGGPSSGAPPPS-NH2
Compound 15: 
H-Aib-QGTFTSDK(GGSGSG-γGlu-CO(CH2)18-COOH)SKYLDERAAQD
F-Iva-QWLLDGGPSSG-Aib-PPPS
Compound 16:

H-Aib-QGTFTSDK(GGSGSG-γGlu-CO(CH2)18-COOH)SKYLDERAAQD
F-Iva-QWLLDGGPSSG-Aib-PPPS-NH2.

12. The use according to Claim 1, wherein the inflammatory bowel diseases and related intestinal fibrosis include Crohn's disease, colitis, intestinal fibrosis caused by Crohn's disease, and intestinal fibrosis caused by colitis.

13. A composition, comprising the polypeptide compound according to any of Claims 1 to 12.

14. The composition according to Claim 13, wherein the composition is a pharmaceutical composition comprising a pharmaceutically acceptable carrier or excipient.
